(19) Europäisches Patentamt / European Patent Office / Office européen des brevets

(11) **EP 4 489 711 B1**

(12) **EUROPEAN PATENT SPECIFICATION**

(45) Date of publication and mention of the grant of the patent:
**15.04.2026 Bulletin 2026/16**

(21) Application number: **22711458.4**

(22) Date of filing: **09.03.2022**

(51) International Patent Classification (IPC):
**A61F 13/537** *(2006.01)* **A61L 15/42** *(2006.01)*
**A61L 15/60** *(2006.01)*

(52) Cooperative Patent Classification (CPC):
(C-Sets available)
**A61F 13/53713; A61F 13/537; A61F 13/53717;**
**A61F 13/53747; A61F 13/5376; A61L 15/60;**
A61F 2013/530481; A61F 2013/530708;
A61F 2013/530788 (Cont.)

(86) International application number:
**PCT/CN2022/079864**

(87) International publication number:
**WO 2023/168616 (14.09.2023 Gazette 2023/37)**

(54) **ABSORBENT ARTICLE WITH HIGH PERMEABILITY SAP**

ABSORBIERENDER ARTIKEL MIT HOCHPERMEABLEM SAP

ARTICLE ABSORBANT À POLYMÈRE SUPERABSORBANT À PERMÉABILITÉ ÉLEVÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.01.2025 Bulletin 2025/03**

(73) Proprietor: **The Procter & Gamble Company Cincinnati, OH 45202 (US)**

(72) Inventors:
• **KAMPHUS, Juliane**
  **65824 Schwalbach Am Taunus (DE)**
• **YUAN, Yi**
  **Beijing 101312 (CN)**
• **PERI, Andrea**
  **65824 Schwalbach Am Taunus (DE)**
• **DIJAKOV, Natasa**
  **65824 Schwalbach am Taunus (DE)**

(74) Representative: **P&G Patent Germany Procter & Gamble Service GmbH Sulzbacher Straße 40 65824 Schwalbach am Taunus (DE)**

(56) References cited:
WO-A1-2016/134905    WO-A2-2020/205485
US-A1- 2011 125 119    US-A1- 2015 328 358
US-A1- 2021 177 672

(52) Cooperative Patent Classification (CPC): (Cont.)

C-Sets
**A61L 15/60, C08L 33/02**

**Description**

FIELD OF THE INVENTION

The invention relates to absorbent articles for personal hygiene, such as baby diapers and adult incontinence products.

BACKGROUND OF THE INVENTION

Disposable absorbent articles such as diapers comprise an absorbent core disposed between a liquid permeable topsheet on their wearer-facing side and a liquid impermeable backsheet on their garment-facing side. The absorbent core typically comprises an absorbent material disposed within a core wrap. Urine is acquired into the absorbent article through the topsheet and is absorbed by the absorbent material.

Superabsorbent polymers (SAP) are commonly used as absorbent material. While surface cross-linking is known to increase the permeability and swelling kinetics of SAP, it is also known that the different performance profiles of SAP trade one property in respect of the other. Specifically, one of the known trade-off is between permeability and capacity. Increasing the permeability of the SAP can improve the locally accessible capacity at earlier gushes, however this reduces capacity for later gushes and higher loads where capacity is a more important property than permeability.

The superabsorbent polymers are typically in the form of particles mixed with cellulose fibers (the core then being referred to as "fluff pulp" or "airfelt" core). US2011/0125119A1 discloses absorbent cores comprising 10% to 95% by weight of spherical fluid-absorbent polymer particles. Absorbent cores without cellulose fibers ("airfelt-free" cores) have also been proposed. The SAP may be for example enclosed within discrete pockets formed between two substrate layers (see e.g. WO95/11654, Tanzer et al.). It has also been proposed to immobilize SAP particles with a microfibrous thermoplastic adhesive network on a nonwoven substrate (see e.g. WO2008/155699A1, Hundorf et al.).

Most personal absorbent hygiene articles have an acquisition layer directly under the topsheet. The acquisition layer provides fast acquisition of the fluid from the topsheet. In some absorbent articles, a distribution layer is further present between the acquisition layer and the absorbent core. The distribution layer distributes the fluid throughout the plane of the absorbent article to maximize the use of the absorbent material. The distribution layer may be in direct contact with the absorbent core. A distribution layer consisting of cross-linked cellulose fibers has been used in combination with airfelt-free cores, see for example US2008/0312622 (Hundorf). While cross-linked cellulose fibers have a fast speed of acquisition, a distribution layer made of these cross-linked cellulose fibers display poor integrity during use, as the unbonded cellulose fibers can form wet clumps. Such a distribution layer may also be several times thicker than the dry absorbent core.

It is desirable for the overall sensorial performance of the absorbent article to be soft, thin and flexible. A combination of soft nonwoven layers have been recently proposed (see for example US2021/0106471, Yuan) as acquisition-distribution system for an airfelt-free core article. The comparatively reduced volume and lower permeability of these nonwovens can however cause slower acquisition times that might result in increased product leakage in use. Especially airfelt-free absorbent cores may not have sufficient absorption speed to handle a larger amount of liquid in a sufficient manner (as superabsorbent polymer materials typically absorb liquid slower than cellulose fibers, especially when a first gush of liquid wets the article).

In order to maintain leakage performance, it is possible to increase the overall amount of SAP in the core, but this leads to a higher material cost. More recently, it has been suggested to place a lower acquisition and distribution layer between the absorbent core and the backsheet to improve fluid acquisition properties (see WO2021/118904, Grenier). While such a lower acquisition and distribution layer can improve the acquisition time, the performance may still be slower than a diaper with an upper acquisition distribution system comprising cross-linked cellulose fibers as a distribution layer.

There is thus a need for absorbent articles that address the above problems, in particular that have good fluid management properties, are comfortable to use and which do not lose their integrity during use.

SUMMARY OF THE INVENTION

The present invention is, in a first aspect, for an absorbent article comprising a liquid-permeable topsheet, an absorbent core comprising a layer of superabsorbent polymer particles and core wrap, an upper acquisition-distribution system consisting of the layers disposed between the topsheet and the absorbent core, and a liquid-impermeable backsheet. The superabsorbent polymer particles have a Urine Permeability of at least $45.10^{-7}$ $(cm^3.s)/g$, as measured by Urine Permeability Measurement (UPM) Method described herein, and preferably have an Effective Capacity above 23 g/g, wherein the Effective Capacity is measured as described herein. Preferably the superabsorbent polymer particles are not mixed with cellulose fibers.

The upper acquisition-distribution system is defined by all the layers between the topsheet and the absorbent core. The upper acquisition-distribution system comprises one, two or more layers, is free of unbonded cross-linked cellulose fibers and comprises at least one spunlace layer.

The present invention enables absorbent articles without cross-linked cellulose fibers in the upper acquisition-distribution system to have comparable speed of acquisition of the absorbent articles having cross-linked cellulose fibers in the upper ADS. It was surprisingly found that absorbent articles having an airfelt-free cores especially benefit from the use of high permeability SAP, even if this lowers the overall core capacity in comparison with higher capacity SAP.

The SAP used in the invention provide the right balance between capacity and permeability especially in the context of airfelt-free cores comprising an upper acquisition-distribution system that is free of cross-linked cellulose fibers. The upper acquisition-distribution system in particular comprises a spunlace nonwoven, which was found particularly useful to replace cross-linked cellulose fibers.

In further aspects of the invention, the absorbent core may comprise one or more longitudinally-extending channel-forming areas formed by bonding the upper side and the lower side of the core wrap together in material free areas surrounded by the absorbent material. These channel-forming area(s) form three-dimensional channels when wet.

The absorbent article may optionally also comprise a lower acquisition-distribution layer between the absorbent layer and the backsheet. The lower acquisition-distribution layer is preferably disposed between the absorbent core and the backsheet, but it may also be disposed within the absorbent core inside the core wrap.

BRIEF DESCRIPTION OF THE DRAWINGS

While the specification concludes with claims particularly pointing out and distinctly claiming the present invention, it is believed that the same will be better understood from the following description read in conjunction with the accompanying drawings in which:

Fig. 1 is an exemplary absorbent article in the form of a diaper;
Fig. 2 is a transversal cross-section of the diaper of Fig. 1;
Fig. 3A-3C show alternative transversal cross-sections of the diaper comprising a lower acquisition and distribution layer;
Fig. 4 shows a top view of an exemplary absorbent core with the top layer partially removed;
Fig. 5 shows a longitudinal cross-section view of the absorbent core of Fig. 4;
Fig. 6 shows transversal cross-section view of the absorbent core of Fig. 4;
Fig. 7 is a partial cross-sectional side view of a suitable permeability measurement system for conducting the Urine Permeability Measurement Test;
Fig. 8 is a cross-sectional side view of a piston/cylinder assembly for use in conducting the Urine Permeability Measurement Test;
Fig. 9 is a top view of a piston head suitable for use in the piston/cylinder assembly shown in Fig. 7.

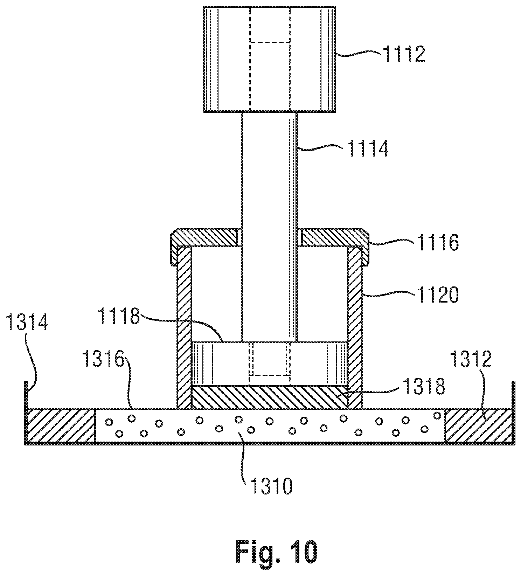

Fig. 10 is a cross-sectional side view of the piston/cylinder assembly of Fig. 8 placed on fritted disc for the swelling phase.

DETAILED DESCRIPTION OF THE INVENTION

Definitions

As used herein, "absorbent articles" refers to personal hygiene devices that absorb and contain body exudates and which are placed against or in proximity to the crotch of a wearer to absorb and contain the various exudates discharged from the body. Absorbent articles include taped diapers and pant diapers (for babies, infants and for adults), absorbent inserts (which are intended to be inserted into an outer cover to form a diaper or pant), feminine care absorbent articles such as sanitary napkins and pantiliners, and the like. As used herein, the term "exudates" includes, but is not limited to, urine, blood, vaginal discharges, sweat and fecal matter. The absorbent articles of the invention are typically disposable and preferably recyclable.

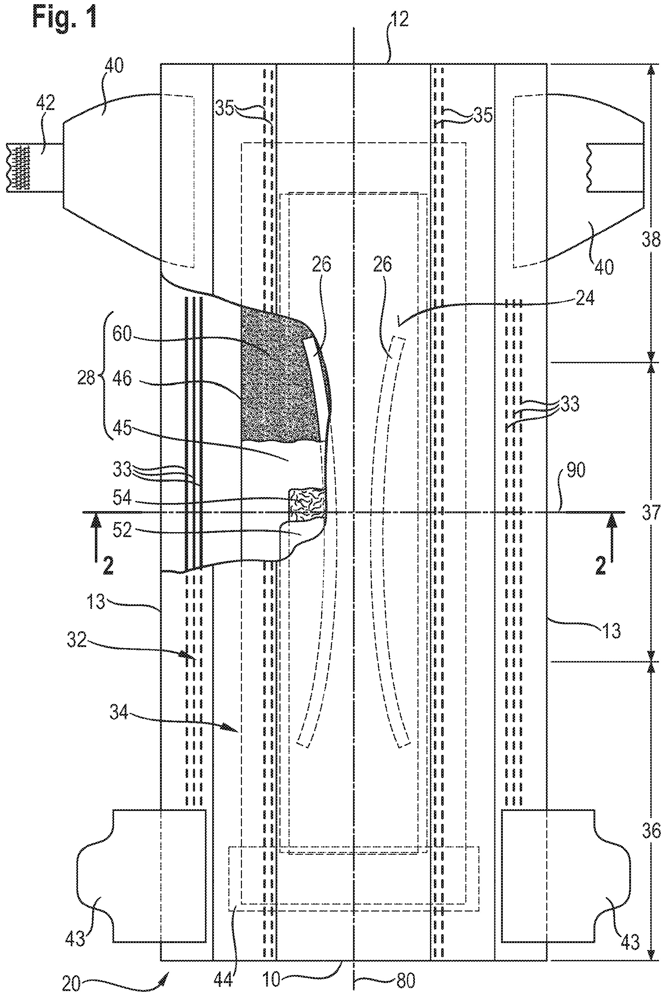

As used herein, "diapers" refers to absorbent articles generally worn by babies, infants and incontinent adults about the lower torso so as to encircle the waist and legs of the wearer and that is specifically adapted to receive and contain urinary and fecal waste. Diapers are typically proposed as taped diapers or pant diapers. Taped diapers have a fastening system (as illustrated in Fig. 1 for example), where the waist opening and leg openings are formed when the diaper is applied onto the wearer by releasably attaching the longitudinal edges of the front waist region and back waist region to each other. In pant diapers, on the other hand the longitudinal edges of the waist regions are attached to each other to form a pre-formed waist opening and leg openings. A pant diaper is placed in position on the wearer by inserting the wearer's legs into the leg openings and sliding the pant diaper into position about the wearer's lower torso. A pant may be pre-formed by any suitable techniques including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

As used herein, the terms "nonwoven", "nonwoven web" and "nonwoven layer" are used interchangeably. Nonwovens are broadly defined as engineered fibrous assemblies, primarily planar, which have been given a designed level of structural integrity by physical and/or chemical means, excluding weaving, knitting or paper making. The fibers may be of natural origin, such as cotton or bamboo fibers, or man-made origin. Synthetic fibers may be selected from the group consisting of polyolefins (such as polyethylene, polypropylene or combinations and mixtures thereof), polyethylene terephthalate (PET), co PET, polylactic acid (PLA), polyhydroxy alkanoid (PHA), or mixtures or combinations thereof. The fibers may be staple fibers (e.g. in carded nonwoven webs/layers) or continuous fibers (e.g. in spunbonded or meltblown nonwoven webs/layers).

Nonwoven webs/layers can be formed by many processes such as meltblowing, spunlaying, solvent spinning, electrospinning, and carding, and the fibers can be consolidated, e.g. by hydroentanglement (in spunlaced nonwoven webs/layers), air-through bonding (using hot air that is blown through the fiber layer in the thickness direction), needle-punching, one or more patterns of bonds and bond impressions created through localized compression and/or application of heat or ultrasonic energy, or a combination thereof. The fibers may, alternatively or in addition, be consolidated by use of a binder. The binder may be provided in the form of binder fibers (which are subsequently molten) or may be provided in liquid, such as a styrene butadiene binder. A liquid binder is provided to the fibers (e.g. by spraying, printing or foam application) and is subsequently cured to solidify. The basis weight of nonwoven fabrics is usually expressed in grams per square meter (g/m$^2$).

The term "spunlace" means a nonwoven wherein the cohesion and the interlacing of the fibers with one another is obtained by means of a plurality of jets of water under pressure passing through a moving fleece or cloth and, like needles, causing the fibers to intermingle with one another. These spunlace are essentially defined by the fact that their consolidation results from hydraulic interlacing. "Spunlace", as used herein, also relates to a nonwoven formed of two or more webs (stratum), which are combined with each other by hydraulic interlacing. The two webs, prior to being combined into one nonwoven by hydraulic interlacing, may have underdone bonding processes, such as heat and/or pressure bonding by using e.g. a patterned calendar roll and an anvil roll to impart a bonding pattern. However, the two webs are combined with each other solely by hydraulic interlacing.

"Monocomponent" refers to fibers formed of a single polymer component or single blend of polymer components, as distinguished from bicomponent or multicomponent fiber.

"Bicomponent" refers to fibers having a cross-section comprising two discrete polymer components, two discrete blends of polymer components, or one discrete polymer component and one discrete blend of polymer components. "Bicomponent fiber" is encompassed within the term "multicomponent fiber." A bicomponent fiber may have an overall cross section divided into two subsections of the differing components of any shape or arrangement, including, for example, concentric core-and-sheath subsections, eccentric core-and-sheath subsections, side-by-side subsections, radial subsections, etc.

"Multicomponent fiber" includes, but is not limited to, "bicomponent fiber." A multicomponent fiber may have an overall cross section divided into subsections of the differing components of any shape or arrangement, including, for example, coaxial subsections, concentric core-and-sheath subsections, eccentric core-and-sheath subsections, side-by-side subsections, islands-in the sea subsection, segmented pie subsections, etc.

Nonwoven materials can be formed by a variety of fiber materials (PP, PE, PET, coPET, bicomponent, and mixture thereof) and, in some cases, the fibers or the nonwovens can be treated to enhance specific fluid handling characteristics, such as fluid permeability or fluid barrier properties.

The term "dtex" as used herein refers to a unit used to indicate the fineness of a filament/fiber. The unit expresses the mass of a filament/fiber in grams per 10,000 meters of length.

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is greater than 90° and the fluid does not spread spontaneously across the surface of the fiber.

"Longitudinal" refers to a direction running substantially perpendicular from a waist edge to an opposing waist edge of the article and generally parallel to the maximum linear dimension of the article (line 80 in Fig. 1). "Transversal" refers to a direction perpendicular to the longitudinal direction (line 90 in Fig. 1).

"Inner" and "outer" refer respectively to the relative location of an element or a surface of an element or group of elements. "Inner" implies the element or surface is oriented towards the inside of the article while "outer" implies the element or surface is oriented towards the outside of the article.

"Body-facing" and "garment-facing" refer respectively to the relative location of the surface of an element or group of elements. "Body-facing" implies the surface is nearer to the wearer during wear than the other surface of the element of group of elements. "Garment-facing" implies the surface is oriented away from the wearer during wear. The garment-facing surface may face another (i.e. other than the wearable article) garment of the wearer, other items, such as the bedding, or the atmosphere.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" encompasses the narrower terms "consisting essential of" which excludes any element, step or ingredient not mentioned which materially affect the way the feature performs its function, and the term "consisting of" which excludes any element, step, or ingredient not specified.

General description of an exemplary diaper

Figure 1 is a plan view of an exemplary diaper 20, in a flat-out state, with portions of the diaper being cut-away to more clearly show the construction of the diaper. This diaper 20 is shown for illustration purpose only as the structure of the present invention may be comprised in a wide variety of diapers or other absorbent articles, such as pant diapers having pre-formed side seams. The side seams of pant articles can be opened by cutting or otherwise, if it is desired to place the pant in a flattened out configuration similar to Fig. 1.

As illustrated in Figure 1-2, the absorbent article comprises a topsheet 24 on its wearer-facing side, a backsheet 25 on its garment-facing side, and an absorbent core 28 between the topsheet 24 and the backsheet 25. The absorbent core 28 comprises at least one layer 60 of superabsorbent polymer particles ("SAP") and a core wrap. The layer of SAP is disposed between a top core wrap layer 45 and a bottom core wrap layer 46.

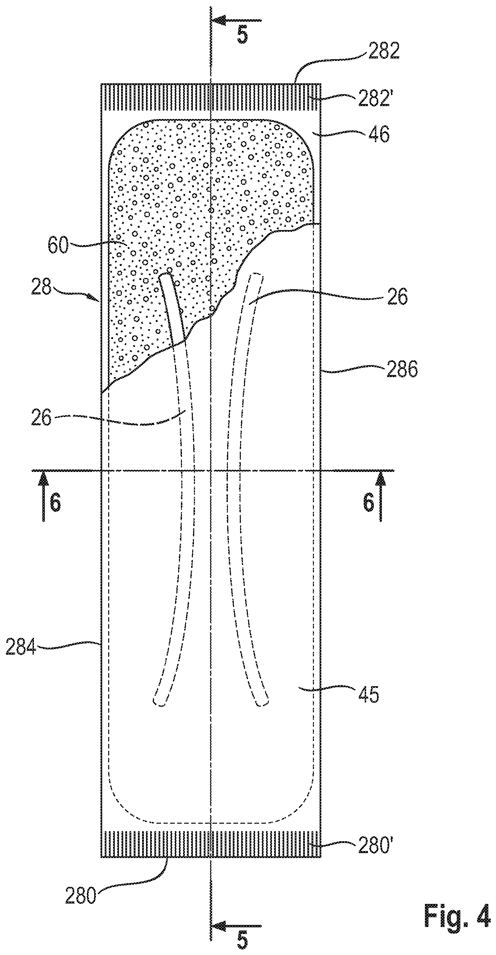

The SAP layer typically has a pre-determined outline (periphery) as considered in the plane formed by the article when flattened-out. This outline may be substantially rectangular as illustrated in Fig. 1, or have another shape such as a sand-hour or dogbone shape. The absorbent core 28 may further comprise at least one longitudinally-oriented channel-forming area 26, which is an area substantially free of SAP defined within the SAP layer. The channel-forming area(s) 26 facilitate(s) the distribution of a fluid along the length of the absorbent article. An exemplary absorbent core construction is discussed in more details below in relation to Figs. 4-6.

The absorbent articles of the invention further comprise an upper acquisition-distribution system. The upper acquisition-distribution system as defined herein consists of all the layers between the topsheet and the absorbent core. The upper acquisition-distribution system typically comprises at least an upper acquisition layer 52 directly underneath the topsheet, and optionally a distribution layer 54 between the acquisition layer and the absorbent core. The upper acquisition-distribution layer may also consist of a single layer, or three layers, or even more layers.

The upper acquisition-distribution system of the invention preferably consists of one or more nonwoven layer(s). The acquisition layer 52 may be for example a surfactant treated, latex bonded, nonwoven acquisition layer. While cross-linked cellulose fibers have been used in the past as distribution layer with airfelt-free absorbent core, these cellulose fibers are loose, that is unbonded and thus do not have the integrity of a nonwoven. It is one object of the invention to remove unbonded cross-linked cellulose fibers without compromising on the speed of acquisition and distribution of a fluid in the article. The upper acquisition-distribution system is thus free of such unbonded cross-linked cellulose fibers. By unbonded, it is meant that the fibers do not form a web that can be manipulated without a supporting layer such as a nonwoven or an airlaid, rather the unbonded fibers form a patch having only a loose integrity and the fibers can be lightly manually separated.

The spunlace layer can be used in the present invention as distribution layer. Suitable spunlace nonwovens comprise absorbent fibers, stiffening fibers and resilient fibers, as for example disclosed in WO2020/205485 (Peri et al.). The spunlace nonwoven may typically comprise from about 20 percent to about 75 percent of absorbent fibers, from about 1 percent to about 50 percent of stiffening fibers, and from about 10 percent to about 50 percent of resilient fibers.

Any suitable absorbent fibers may be utilized. Some conventional absorbent fibers include cotton, rayon or regenerated cellulose or combinations thereof. In one example, the absorbent fibers may comprise viscose cellulose fibers. The absorbing fibers may comprise staple length fibers. The staple length of the absorbing fibers can be in the range of about 20 mm to about 100 mm, or about 30 mm to about 50 mm or about 35 mm to about 45 mm. As noted previously, in addition to absorbent fibers, the spunlace of the invention may also comprise stiffening fibers. Stiffening fibers may be utilized to help provide structural integrity to the nonwoven. The stiffening fibers can help increase structural integrity of the nonwoven in a machine direction and in a cross machine direction which can facilitate web manipulation during processing of the nonwoven for incorporation into a disposable absorbent article. Any suitable stiffening fiber may be utilized. Some examples of suitable stiffening fibers include bi-component fibers comprising polyethylene and polyethylene terephthalate components or polyethylene terephthalate and co-polyethylene terephthalate components. The components of the bi-component fiber may be arranged in a core sheath arrangement, a side by side arrangement, an eccentric core sheath arrangement, a trilobal arrangement, or the like. In one specific example, the stiffening fibers may comprise bi-component fibers having polyethylene/ polyethylene terephthalate components arranged in a concentric, core - sheath arrangement where the polyethylene is the sheath. As another example, mono-component fibers may be utilized, and the constituent material of the mono-component may comprise polypropylene or polylactic acid (PLA). It is worth noting that these components, e.g. polypropylene and polylactic acid can also be utilized in bi-component fibers as well.

The resilient fibers help the spunlace to maintain its permeability and cushion properties. Suitable fibers that may be utilized include in particular hollow fibers, spiral fibers, and/or hollow spiral fibers. For example, the resilient fibers can have a linear density of about 4 dtex to about 12 dtex, from about 6 dtex to about 11 dtex, or from about 8 dtex to about 10 dtex, specifically reciting all values within these ranges and any ranges created thereby. In one specific example, the resilient fibers may comprise a linear density of about 10 dtex hollow spiral (HS) polyethylene terephthalate fibers. In another specific example, the resilient fibers may comprise 6.7 dtex round polyethylene terephthalate fibers.

As illustrated in Fig. 1, the absorbent article, whether a taped diaper or a pant diaper, can be notionally divided in a front waist region 36, a back waist region 38 opposed to the first waist region 36, and a crotch region 37 located between the front waist region 36 and the back waist region 38. The crotch region, the front waist region and the back waist region are hereby defined as delimiting each one third of the length of the absorbent article along the longitudinal centerline 80. The longitudinal centerline 80 is the imaginary line separating the diaper along its length in two equal right and left halves. The transversal centerline 90 is the imaginary line perpendicular to the longitudinal centerline 80 in the plane of the flattened-out diaper and going through the middle of the length of the diaper. The periphery of the diaper 20 is defined by the outer edges of the diaper. The longitudinal edges 13 of the diaper may run generally parallel to the longitudinal centerline 80 of the diaper 20 and the front waist edge 10 and the back waist edge 12 typically run generally parallel to the transversal centerline 90 of the diaper 20. However, these article edges do not need to be straight, at they may be curved to better fit the wearer.

Further, the absorbent article may comprise other optional but conventional elements, which are not represented for simplicity, such as a back waist elastic feature, a front waist elastic feature, a lotion applied onto the body-facing surface of the topsheet, or a urine indicator disposed on the inner side of the backsheet that changes color when contacted with urine.

The topsheet 24, the backsheet 25, and the absorbent core 28 may be assembled in a variety of well-known configurations, in particular by gluing, heat embossing, ultrasonic bonding or combinations thereof. Exemplary diaper configurations are described generally in US3,860,003; US5,221,274; US5,554,145; US5,569,234; US5,580,411; and US6,004,306.

The topsheet 24 is the part of the absorbent article that is in contact with the wearer's skin. At least a portion of, or all of, the topsheet is liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polypropylene or bicomponent PE/PP fibers or mixtures thereof), or a combination of natural and synthetic fibers. The topsheet may have one or more layers. The topsheet may be apertured or non-apertured, and may have any suitable three-dimensional features, and/or may have a plurality of embossments (e.g., a bond pattern). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The backsheet 25 is generally that portion of the absorbent article 20 that constitutes all or a part of the garment-facing surface of the absorbent article. The backsheet 25 may be joined at least partially to the topsheet 24, the absorbent core 28, or a lower acquisition and distribution layer 56 if present, by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable.

The backsheet typically comprises a thin impermeable plastic film, usually a thermoplastic film having a thickness of about 0.01 mm to about 0.05 mm. The backsheet material may be breathable, which permit vapors to escape from the absorbent article, while still preventing, or at least inhibiting, bodily exudates from passing through the backsheet. A breathable backsheet may have a Water Vapor Transmission Rate (WVTR) of from 1,000 to 15,000 $g/m^2/24h$, or from 1,000 to 10,000 $g/m^2/24h$, or from 1,500 to 10,000 $g/m^2/24h$ as measured using a PERMATRAN-W Model 101K (available from Mocon, Inc., Minneapolis, MN) or equivalent, according to Nonwovens Standard Procedure NWSP 70.4.R0(15) with the following specifications: experiments were carried out in a lab controlled at 23 °C $\pm$ 2 C° and 50 %RH $\pm$ 2 %RH and the instrument cells heated to 37.8°C (100°F).

The backsheet 25 may also comprise a backsheet outer cover nonwoven (not represented). The backsheet outer cover nonwoven is typically a thin nonwoven material that is joined to the outer surface of the backsheet film. The outer cover nonwoven may thus form the garment-facing surface of the backsheet. The backsheet outer cover nonwoven may comprise a bond pattern, apertures, and/or three-dimensional features, and may improve the feel of the backsheet.

The absorbent article 20 may also comprise inner barrier leg cuffs 34 and outer leg cuffs 32, as is known in the art. The inner barrier cuffs 34 can extend upwards from the surface of the article to provide retention of the waste, while the outer cuffs are typically formed in the plane of the chassis of the article as defined by topsheet and backsheet. These cuffs are preferably elasticized, as is known in the art, for example using elastic threads 33, 35 as represented in the Figures.

Moreover, the absorbent article may comprise a fastening system, such as an adhesive fastening system or a hook and loop fastening member, which can comprise tape tabs 42 disposed on back ears 40, such as adhesive tape tabs or tape tabs comprising hook elements, cooperating with a landing zone 44 (e.g. a nonwoven web providing loops in a hook and loop fastening system). While taped diapers typically comprise back ears 40, and front ears 43, these are typically not present in pant-type absorbent articles having pre-formed side seams.

The front and/or back ears may be separate components attached to the absorbent article or may instead be continuous with portions of the topsheet and/or backsheet such that these portions form all or a part of the front and/or back ears 40, 43. Also combinations of the aforementioned are possible, such that the front and/or back ears 40, 43 are formed by portions of the topsheet and/or backsheet while additional materials are attached to form the overall front and/or back ears 40, 43. The front and/or back ears may be elastic or non-elastic. Also, the front ears 40 may be applied as separate components attached to the absorbent article while the back ears (or parts thereof) may be continuous with portions of the backsheet and/or topsheet - or vice versa.

Absorbent core 28

The absorbent core comprises at least one layer of superabsorbent polymer particles (SAP). SAP are water-insoluble, water-swellable polymers capable of absorbing large quantities of fluids, as is known in the art. The term "superabsorbent polymer" refers herein to absorbent materials, typically cross-linked polymeric materials, that can absorb at least 10 times their weight of an aqueous 0.9% saline solution as measured using the Centrifuge Retention Capacity (CRC) test as indicated in EDANA method NWSP 241.0.R2 (19). The SAP may in particular have a CRC value of more than 20 g/g, or more than 24 g/g, or of from 20 g/g to 50 g/g, or from 20 g/g to 40 g/g, or 24 g/g to 35 g/g. The SAP used in the invention are described in greater details in a section further below.

The SAP are typically immobilized within a core wrap comprising a top core wrap layer and bottom core wrap layer, so that the absorbent core can be easily integrated with the rest of the chassis of the absorbent article in a converting line.

While in the prior art superabsorbent polymer particles are often mixed with cellulose fibers (airfelt cores), the absorbent cores of the invention comprise at least one layer of SAP, wherein the SAP particles are not mixed with cellulose fibers. The resulting layer of absorbent material may thus have a reduced thickness in the dry state, compared to conventional airfelt-based absorbent cores. The reduced thickness helps to improve the fit and comfort of the absorbent article for the wearer. The absorbent cores of the invention may be preferably completely free of unbonded cellulose fibers (however some cellulose fibers may be present in bonded form in a nonwoven or tissue layer, such as a spunlace layer). Many absorbent core designs may be used in the present invention. For example some cellulose fibers may be present in a core wrap if the core wrap comprises a tissue paper layer. So while not preferred, it is also not excluded that the absorbent core may also comprise a separate layer of cellulose fibers or a separate airfelt/SAP mixed layer, as long as it is distinct from the layer of SAP having the Urine Permeability of at least $45.10^{-7}$ $(cm^3.s)/g$.

Various absorbent core designs comprising a layer of SAP free of cellulose fibers have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US2006/024433 (Blessing), US2008/0312617 and US2010/0051166A1 (both to Hundorf et al.) may be used.

The layer of SAP may be typically deposited on at least one layer of the absorbent core serving as substrate, such as the bottom core wrap layer or top core wrap layer. In the SAP-printing process as described in US2008/312,622A1 (Hundorf), a continuous layer of SAP is obtained by depositing SAP on each of the core wrap layers in a pattern having absorbent material land areas separated by absorbent material-free junction areas. The absorbent material land areas of the first layer correspond substantially to the absorbent material-free junction areas of the second layer and vice versa, so that a continuous layer of SAP is obtained when the two discontinuous layers are combined.

The absorbent core may comprise one or more glue layers, in particular an auxiliary glue applied between the internal surface of one or both of the core wrap layers and the SAP layer to adhesively immobilize the SAP within the core wrap. A micro-fibrous thermoplastic adhesive net may also be used in airfelt-free cores as described in the above Hundorf references to immobilize the SAP. These adhesives are not represented in the Figures for simplicity.

Other core constructions, for example comprising a high loft nonwoven substrate such as a carded nonwoven layer, having a porous structure into which SAP particles have been deposited, may also be used in present disclosure.

The layer of SAP may be deposited as a continuous layer within the core wrap. The layer of SAP may also be present discontinuously, for example, as individual pockets or stripes of absorbent material enclosed within the core wrap and separated from each other by material-free junction areas.

The basis weight (amount deposited per unit of surface) of the superabsorbent material may also be varied to create a profiled distribution of superabsorbent material, in particular in the longitudinal direction to provide more absorbency in the crotch portion of the article , but also in the transversal direction, or both directions of the core.

The core wrap is formed by one or two substrate layers that sandwiches the SAP particles and a least partially immobilizes the SAP particles so that the absorbent core keeps its integrity. The top core wrap layer 45 and the bottom core wrap layer 46 are also referred to in the art as core cover and dusting layer respectively. These core wrap layers are typically low basis weight nonwoven (typically less than 20 gsm, in particular from 8 gsm to 14 gsm), and may be in particular SMS nonwoven (Spunbond-Meltblown-Spunbond laminate), as is known in the art. The upper and lower core wrap layers may be any material capable of containing and providing a support for the absorbent material.

The core wrap layers may be made of the same or different materials, i.e. two nonwoven webs which have the same of different properties e.g. in a c-wrap configuration. The core wrap may also be made of a single, continuous nonwoven web, which is wrapped around the layer of absorbent material as this may simplify construction and comprising a single longitudinal seal, in this case the top core wrap and the bottom core wrap layers are made of the same web material.

Figs. 4-6 show an exemplary absorbent comprising a top core wrap layer 45 oriented towards the topsheet, a bottom core wrap layer 46 oriented towards the backsheet, and a layer of SAP 60 between the two core wrap layers. The absorbent core represented has two longitudinal edges 284, 286 and a front and back transversal edges 280, 282 formed by the core wrap.

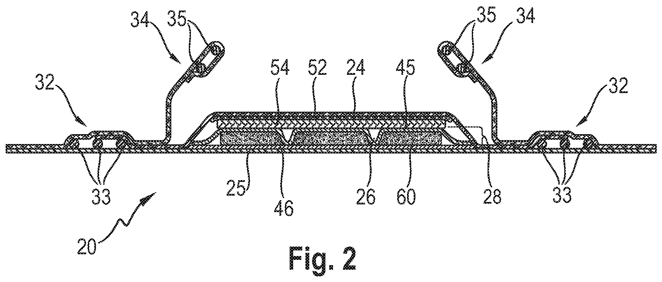

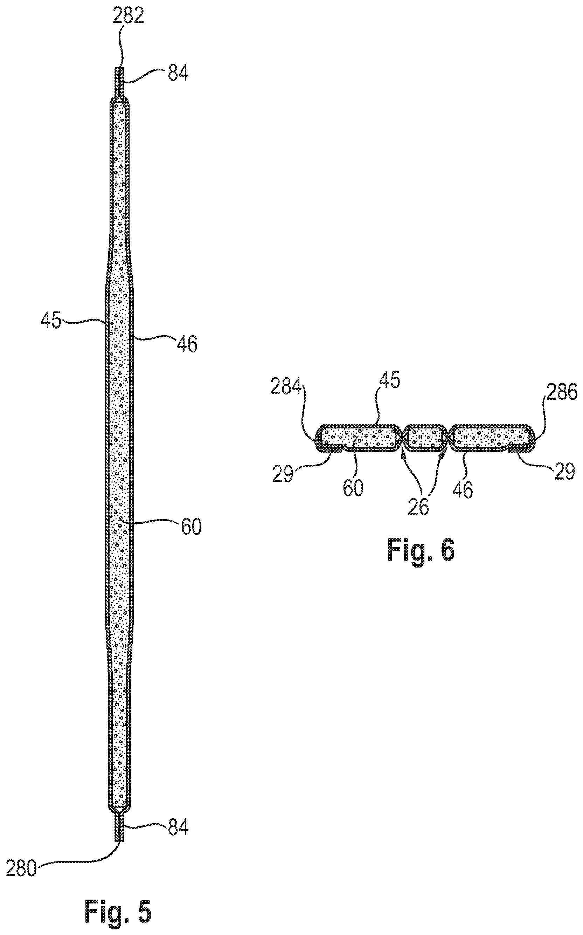

The core wrap layers are preferably bonded longitudinally by one or more longitudinal core wrap bond(s) 29 to prevent the absorbent material from escaping sideways from the absorbent core. The core wrap layers may also be optionally bonded transversally at the front and the back of the absorbent core by one or more transversal core wrap bond 84. The core wrap layers may be bonded face to face, at least longitudinally as represented in Fig. 2, but other bonding configurations are possible, in particular a C-wrap configuration where one of the top or bottom core wrap layer is larger than the other, so that flaps can be folded around the absorbent material and attached to the other core wrap layer as illustrated in Fig. 6. Transversally extending portions of top core wrap layer 45 may be around over the longitudinal edges 284, 286 of the core and be externally bonded to the bottom wrap layer 46, that these flap portions are positioned on the garment-facing surface of the absorbent core. Alternatively, flap portions at and adjacent to the longitudinal edges of the bottom core wrap layer may be folded around the side edges of the core, such that these flap portions are positioned on the body-facing surface of the absorbent core.

The top core wrap layer 45 and the bottom core wrap layer 46 typically at least partially or fully enclose the layer of SAP 60, providing for dry and wet immobilization of the absorbent material. Additionally, the SAP layer may at least partially be immobilized on the top core wrap layer 45 and/or on the bottom core wrap layer 46 (and/or on the lower acquisition distribution layer 56 if present within the core wrap), by a hotmelt adhesive applied between the substrate layer and the SAP and/or by a thermoplastic fibrous network applied on layer of the SAP.

The absorbent core 28 optionally comprises at least one channel-forming area 26, where substantially no absorbent material is present (possibly some superabsorbent particles may be deposited accidental during core-making). The channel-forming area preferably does not extend to any of the side of the absorbent layer, and thus is completely surrounded by the absorbent material. The channel-forming area is typically elongated in the longitudinal direction, having a longitudinal length of from 20% and 80%, or from 20% to 70%, or from 30% to 60%, of the longitudinal length of the layer of SAP 60 (longitudinal length means the length as measured projected on the longitudinal axis). The absorbent core may typically comprise a pair of channel-forming areas disposed symmetrically on each side of the longitudinal axis 80, wherein these channel-forming areas may be straight, curved, or combinations thereof. Such a pair of channel-forming areas may be disconnected, as illustrated in Fig. 4. The channel-forming areas may also be connected, for example at one or both their extremities to form a U or O shape. Examples of channel-forming areas are disclosed in further details in WO2012170778A1, WO2012170781 (Kreuzer et al.).

The top core wrap layer 45 and bottom core wrap layer 46 are preferably bonded to each other through at least a portion of the length of the channel-forming area(s). This bond provides for structural integrity of the channels in dry and wet state. Any known bonding techniques known in the art may be used to provide for this bond, in particular one selected from adhesive bonding, thermo bonding, mechanical bonding, ultrasonic bonding, or any combinations thereof. An adhesive may be for example applied in the areas of the channels on the inner side of the top side and/or the inner side of the bottom side of the core wrap, typically by slot glue application or any other means, followed by the application of pressure in the areas of the channels to provide a good adhesive bonding in these areas. Exemplary patent disclosures of such adhesive bonding processes can be found for an airfelt or airfelt-free absorbent cores in WO2012/170798A1 (Jackels et al.), EP2,905,000 (Jackels et al.) and EP2,905,001 (Armstrong-Ostle et al.).

Other bonding such as thermo bonding, mechanical bonding, ultrasonic bonding can also be used as additional bonding or as an alternative bonding. For example, an adhesive bonding may be reinforced by a thermo bonding, mechanical bonding or ultra-sonic bonding. Such thermo, mechanical or ultrasonic bonding can be applied on the channels through the external sides of the core wrap layers.

Typically, the channel bonds may generally have the same outline and shape as the channel-forming areas 26 in which they are contained, but may be slightly smaller to allow for a safety margin (e.g. by a few mm) as some deviations from the optimal registration may happen during high speed process. The channel-forming area(s) form three-dimensional channel(s) during use as the rest of the absorbent layer absorbent core absorbs a fluid and swells. The channel-forming area(s) are optional in the present invention.

The total amount of SAP present in the absorbent core is adapted to the need of the expected wearer of the article. Diapers for newborns require less SAP than infant or adult heavy incontinence diapers. The amount of SAP in the core may be for example comprised from about 2 g to 50 g, in particular from 5 g to 40 g for typical enfant diapers. The average SAP basis weight within the absorbent core may be for example of at least 50, 100, 200, 300, 400, 500 $g/m^2$ or more, or from 200 $g/m^2$ to 400 $g/m^2$. The average SAP basis weight is the total amount of SAP in the core divided by the area delimited by the periphery of the SAP layer (including any channel-forming areas if present).

Superabsorbent particles 60

Superabsorbent polymers (SAP) are typically water-insoluble but water-swellable cross-linked polymers capable of absorbing large quantities of fluids. SAP are typically in particulate form so as to be flowable in the dry state which facilitate its deposition on a substrate. Typical SAP are made of polyacrylate polymers, however it is not excluded that other polymer materials may also be used.

The absorbent core of the invention comprises at least one layer of superabsorbent polymer particles that have a Urine Permeability Measurement ("UPM") of more than 45 UPM units, where 1 UPM unit is $1 \times 10^{-7}$ $(cm^3.s)/g$. The UPM value is advantageously at least 50 UPM units, in particular at least 55 UPM units or at least 60 UPM units. The SAP may preferably have UPM in the range of from 55 to 90 UPM units, in particular from 55 to 80 UPM units, more particularly in the range of from 60 to 75 UPM units, where 1 UPM unit is $1 \times 10^{-7}$ $(cm^3.s)/g$. The UPM value is measured according to the Urine Permeability Measurement (UPM) Method described herein. The UPM method measures the flow resistance of a preswollen layer of superabsorbent polymer particles, i.e. the flow resistance is measured after loading the SAP with saline solution. Therefore, such superabsorbent polymer particles having a high UPM value exhibit a high permeability when a significant volume of the absorbent article is already wetted by the liquid exudates.

Unless otherwise indicated, the values indicated herein to qualify the SAP (e.g. SAP UPM, EFFC, T20 ...) refer to the properties of SAP forming the layer considered. For example, if two distinct layers of SAP are comprised in the absorbent core, and the SAP used are different in each layer, at least one of these should have the UPM value claimed. If the SAP layer is formed by combining two intermediate layers of SAP layer (as known for printed SAP technology, see below), and these SAP are no longer distinct but form a unitary layer, the SAP values refer of the whole of the SAP forming the combined SAP layer.

The superabsorbent polymer particles preferably have an Effective Capacity (EFFC) above 23 g/g, more preferably in the range of from 23 g/g to 30 g/g, in particular in the range of from 23.5 g/g to 29 g/g, or 24 g/g to 28 g/g, or 24.5 g/g to 27 g/g. The Effective Capacity (EFFC) is calculated via the formula: EFFC = (CRC+AAP)/2. The Centrifuge Retention Capacity (CRC) is measured according to the Centrifuge Retention Capacity (CRC) test method as set out herein, and the Absorption Against Pressure (AAP) is measured according to the Absorption Against Pressure (AAP) test method as set out herein. The SAP of the invention may have an AAP of at least 22 g/g or from 23 to 24.5 g/g or from 24.0 to 24.5 g/g.

The SAP particles may further have a bulk density of at least 0.5 g/ml, measured according to the Bulk Density test method.

SAP having the desired properties can be ordered from well-known SAP manufacturers, independently of the method of fabrication. These SAP used in the invention can be typically obtained by surface cross-linking precursor SAP. Suitable precursor SAP particles may for example be obtained as described in WO2015/041,784A1 or in EP2,535,027A1, or from inverse phase suspension polymerizations as described in US4,340,706 and US5,849,816, or from spray- or other gas-phase dispersion polymerizations as described in US2009/0192035, US2009/0258994 and US2010/0068520. In some embodiments, suitable precursor superabsorbent polymer particles may be obtained by production processes as is more particularly described from page 12, line 23 to page 20, line 27 of WO 2006/083584.

The precursor water-absorbing polymer particles are typically internally crosslinked, i.e., the polymerization is carried out in the presence of compounds having two or more polymerizable groups which can be free-radically copolymerized into the polymer network. Useful crosslinkers ii) may include for example ethylene glycol dimethacrylate, diethylene glycol diacrylate, allyl methacrylate, trimethylolpropane triacrylate, triallylamine, tetraallyloxyethane as described in EP-A 530 438, di- and triacrylates as described in EP-A 547 847, EP-A 559 476, EP-A 632 068, WO 93/21237, WO 03/104299, WO 03/104300, WO 03/104301 and in the DE-A 103 31 450, mixed acrylates which, as well as acrylate groups, comprise further ethylenically unsaturated groups, as described in DE-A 103 31 456 and DE-A 103 55 401, or crosslinker mixtures as described for example in DE-A 195 43 368, DE-A 196 46 484, WO 90/15830 and WO 02/32962.

Preferably, the internal crosslinkers ii) are diacrylated, dimethacrylated, triacrylated or trimethacrylated multiply ethoxylated and/or propoxylated glycerols. Di- and/or triacrylates of 3- to 10-tuply ethoxylated glycerol are particularly advantageous. More preferably, the crosslinkers ii) are di- or triacrylates of 1- to 5-tuply ethoxylated and/or propoxylated glycerol. Preferably, the internal crosslinkers comprise acrylate or acrylamide groups.

Detailed examples of making SAP according to the invention by surface cross-linking is provided in the section below entitled "Method of making the SAP".

The SAP used in the invention may be surface crosslinked. Commonly applied surface cross-linkers are thermally activated surface cross-linkers. The term "thermally activated surface cross-linkers" refers to surface cross-linkers, which only react upon exposure to increased temperatures, typically around 150 °C. Thermally activated surface cross-linkers known in the prior art are e.g. di- or polyfunctional agents that are capable of building additional cross-links between the polymer chains of the precursor superabsorbent polymer particles. Other thermally activated surface cross-linkers include, e.g., di- or polyhydric alcohols, or derivatives thereof, capable of forming di- or polyhydric alcohols. Representatives of such agents are alkylene carbonates, ketales, and di- or polyglycidlyethers. Moreover, (poly)glycidyl ethers, haloepoxy compounds, polyaldehydes, polyoles and polyamines are also well known thermally activated surface cross-linkers. The cross-linking is based on a reaction between the functional groups comprised by the precursor superabsorbent polymer particle, for example, an esterification reaction between a carboxyl group (comprised by the polymer) and a hydroxyl group (comprised by the surface crosslinker).

In general, the surface cross-linking agent is applied on the surface of the precursor superabsorbent polymer particles. Therefore, the reaction preferably takes place on the surface of the precursor superabsorbent polymer particles, which results in improved cross-linking on the surface of the particles while not substantially affecting the core of the particles. Thereby, the surface of the superabsorbent polymer particles becomes stiffer.

The SAP of this invention may for example be surface-crosslinked using alkylene carbonates as surface-crosslinking agent, preferably using a surface-crosslinking solution comprising deionized water. Preferably, the application of surface-crosslinking solution is applied via a stray-coating. The heat treatment of the SAP of this invention may be done at elevated temperature, preferably more than 185°C, more preferably from 190°C to 205°C.

The SAP of this invention is preferably classified after the surface-crosslinking and the subsequent heat treatment, e.g. using a sieving step. Preferably, the SAP of this invention is sieved to a particle size in the range of 710 $\mu$m to 45 $\mu$m, as measured by EDANA NWSP 220.0R2 (19).

The SAP K(t) Test Method described below is also useful to determine other SAP parameters, which may also be advantageously used in the present invention. The SAP used in the core may advantageously have a time to reach an uptake of 20 g/g (SAP T20) of less than 220 s as measured by the SAP K(t) test method. The SAP may in particular have a SAP T20 of from 100 s to 220 s. The SAP T20 values may be less than 200 s, or less than 180 s, or less than 160 s. The time T20 may also be of at least of 100 s, 104 s, 120 s or 140 s, and any combinations of these upper and lower values to form a range, e.g. of from 100 s to 200 s.

The uptake of the SAP at 20 min (U20) may be in particular of at least 22 g/g, or at least 24 g/g, or at least 28 g/g or at least 30 g/g, or of from 28 g/g to 60 g/g, or of from 30 g/g to 50 g/g, or of from 30 g/g to 40 g/g as measured according to the SAP K(t) test method disclosed herein. The SAP may have an effective permeability at 20 minutes (SAP K20) of at least 1 $\times$ 10$^{-8}$ cm$^2$, or at least 2 $\times$ 10$^{-8}$ cm$^2$, or at least 2.5$\cdot \times$ 10$^{-8}$ cm$^2$, or of 3 $\times$ 10$^{-8}$ cm$^2$ to 1 $\times$ 10$^{-7}$ cm$^2$, or of 2 $\times$ 10$^{-8}$ cm$^2$ to 7 $\times$ 10$^{-8}$ cm$^2$, or of 2.5$\cdot \times$ 10$^{-8}$ to 5 $\times$ 10$^{-8}$ cm$^2$ as measured according to the SAP K(t) test method.

The SAP may also have a ratio between the minimum effective permeability and the permeability at 20 minutes (SAP Kmin/SAP K20 ratio) of more than 0.75, or more than 0.8 or more than 0.85 as measured according to the SAP K(t) test method. In such embodiments the transient gel blocking is minimum and the liquid exudates are able to travel fast through the void spaces present between the particles throughout all the swelling process and especially in the initial part of the swelling phase which is the most critical for the first gush.

The SAP of the invention are typically selected from polyacrylates and polyacrylic acid polymers that are internally and surface cross-linked. The superabsorbent polymers can be internally cross-linked, i.e. the polymerization is carried out in the presence of compounds having two or more polymerizable groups which can be free-radically copolymerized into the polymer network. Exemplary superabsorbent polymer particles of the prior art are for example described in WO2006/083584, WO2007/047598, WO2007/046052, WO2009/155265, WO2009/155264. Preferably, the SAP particles comprise crosslinked polymers of polyacrylic acids or their salts or polyacrylates or derivatives thereof.

The SAP particles may be relatively small (under 1 mm in their longest dimension) in their dry state and may be roughly circular in shape, but granules, fibers, flakes, spheres, powders, platelets and other shapes and forms are also known to persons skilled in the art. Typically, the SAP may be in the form of spherical-like particles.

At least a portion of the SAP particles may be agglomerated, as e.g. taught in EP3,391,961A1 (Kamphus, P&G). The agglomerated superabsorbent polymer particles may be obtained by various methods. Agglomerated particles may be for example obtained by aggregating the precursor particles with an interparticle crosslinking agent reacted with the polymer material of the precursor particles to form crosslink bonds between the precursor particles have been for example disclosed in US5,300,565, US5,180,622, (both to Berg), US5,149,334, US5,102,597 (both to Roe), US5,492,962 (Lahrman). Other ways to obtain agglomerated SAP particles are for example described in EP3056521B1 (Kim et al.), EP1512712B1 (Koji et al.), US10414876B2 (Jang et al.), US7429009B2 (Nagasawa et al), EP220224911 (Higashimoto et al), EP2011803B1 (Handa et al).

Agglomerated superabsorbent polymer particles may also be obtained by a method comprising the steps of providing superabsorbent polymer particles and mixing the superabsorbent polymer particles with a solution comprising water and a multivalent salt having a valence of three or higher. This method is further disclosed EP2,944,376A1. The superabsorbent polymer particles may in particular comprise at least 5%, or at least 10%, or at least 20%, or at least 30%, or at least 40%, or at least 50% of agglomerated SAP by weight of the SAP. A method for producing agglomerated superabsorbent polymer particles comprising clay platelets with edge modification and/or surface modification is disclosed in EP3391963.

The surface of the SAP particles may be coated. The SAP may also comprise surface and/or edge modified clay platelets. Preferably, the clay platelets are montmorillonite, hectorite, laponite or mixtures thereof. Preferably, the clay platelets are laponite. The SAP may comprise from 0.1 to 5% by weight of clay platelets with modified surfaces and/or edges compared to the weight of the precursor superabsorbent polymer particles.

Upper acquisition and distribution system ("upper ADS")

Referring to Figs. 1 and 2, the absorbent article 20 of the present disclosure comprises an upper acquisition-distribution system (referred herein as "upper ADS"). The ADS encompasses all the layers 52, 54 disposed between the topsheet 24 and the absorbent core 28 (one or more layers). The upper ADS can quickly acquire the body fluid such as urine and distribute it to the absorbent core 28 in an efficient manner. The upper ADS may comprise a single layer or it may have two or more layers, which may form a unitary structure or may remain as discrete layers which may be attached to each other by, for example, thermal bonding, adhesive bonding or a combination thereof. A unitary structure herein intends to mean that although it may be formed by several sub-layers that have distinct properties and/or compositions from one another, they are somehow intermixed at the boundary region so that, instead of a definite boundary between sub-layers, it would be possible to identify a region where the different sub-layers transition one into the other. Such a unitary structure is typically built by forming the various sub-layers one on top of the other in a continuous manner, for example using air laid or wet laid deposition. Typically, there is no adhesive used between the sub-layers of the unitary material. However, in some cases, adhesives and/or binders can be present although typically in a lower amount that in multilayer materials formed by separate layers.

As indicated previously, cross-linked cellulose fibers have been used in the art to form a distribution layer deposited on a latex bonded nonwoven acquisition layer. Such cross-linked cellulose fibers are unbonded with each other, and contrary to nonwovens or airlaid layers, they do not have an inherent integrity, which causes the cross-linked cellulose fibers to form clumps during use, which reduce the benefits of such a distribution layer.

According to the present invention, the upper ADS is free of unbonded cross-linked cellulose fibers. The upper ADS preferably consists of one or more nonwoven layers. The upper ADS may comprise cellulose fibers formed into a web such as in an airlaid nonwoven layer that comprise a mix of cellulose fibers and synthetic fibers.

The upper ADS may comprise two layers: a first layer 52 directly under the topsheet and second layer 54 between the first layer and the absorbent core. Examples of suitable dual layer ADS are disclosed in US20210106471 A1 (Yuan et al., P&G) disclosing a first layer which is a carded air-through nonwoven and a second layer being an airlaid nonwoven. Without being bound by theory, the thermoplastic fibers enhance structural integrity of the fluid distribution layer while also providing for a more open structure. The cellulose fibers provide liquid storage capability and provide a springy open structure that enables quick recovery of the fluid distribution layer to enable readiness for multiple insults.

The first layer 52 may be an acquisition nonwoven layer as known in the art, such as an air-through bonded carded nonwoven, for example having a basis weight of from 20 gsm to 100 gsm, in particular from 30 gsm to 80 gsm. Such acquisition layer is typically made of synthetic fibers that have been hydrophillically treated with a surfactant. The acquisition layer 52 may also be a latex-bonded, hydrophillically-treated nonwoven.

The second layer 54 of the upper ADS, if present, may be selected from a variety of nonwoven materials, for example an airlaid nonwoven comprising a mix of cellulose and synthetic fibers which have been combined to form a unitary nonwoven web.

The second layer 54 may in particular comprise or consists of a spunlace nonwoven, which have been found to provide good fluid handling, integrity and flexibility. Suitable spunlaces may have a basis weight ranging from about 40 grams per square meter (gsm) to about 200 gsm, preferably from about 70 gsm to about 120 gsm. The spunlace typically comprises a plurality of absorbent fibers, a plurality of stiffening fibers and a plurality of resilient fibers which have been integrated in a spunlace. The spunlace may comprise:

- from about 20 percent to about 75 percent of absorbent fibers;
- from about 1 percent to about 50 percent of stiffening fibers;
- from about 10 percent to about 50 percent of resilient fibers.

Examples of suitable spunlace are for example disclosed in WO2020/205,485A2 (Peri et al., P&G)

Other materials for the second layer are still possible, for example spunbond nonwoven or spunbond-meltblown-spunbond ('SMS") nonwoven. SMS can mean a three layer, 'sms' nonwoven materials, a five layer 'ssmms' nonwoven materials, or any reasonable variation thereof wherein the lower case letters designate individual layers and the upper case letters designate the compilation of similar, adjacent layers.

The upper ADS of the present invention is typically free of superabsorbent material. The upper ADS for the present invention may have an overall basis weight in the range of from about 20 gsm to about 220 gsm, in particular from about 40 gsm to about 160 gsm, as calculated on average for the whole area of the upper ADS. In some embodiments, the upper ADS according to the present invention comprises less than about 40 gsm of cellulose fibers. A basis weight of the first layer may be determined to balance acquisition-distribution performance and the overall thickness of the absorbent article. When the upper ADS comprises an acquisition layer 52 and a distribution layer 54, the distribution layer may typically have about the same, or a higher, basis weight than the acquisition layer. Basis weight can be calculated as usual by dividing the weight of the layer by its area.

Lower acquisition and distribution layer (lower "ADL") 56

The articles of the invention may optionally comprise an acquisition-distribution layer between the layer of SAP and the backsheet (referred herein as "lower ADL"). The use of a lower ADL is optional to provide additional temporary storage while minimizing or preventing dampness perception from the outside surface of the absorbent article.

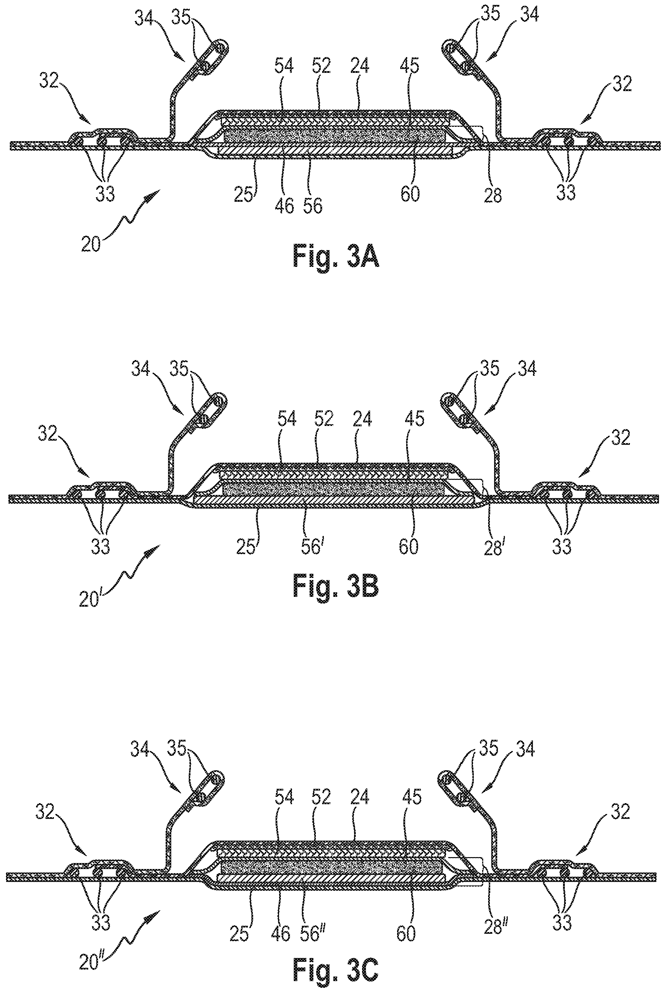

The lower ADL 56 is typically provided between the absorbent core 28 and the backsheet as represented in Fig. 3A. In other words, the lower ADL is typically disposed between the bottom core wrap layer 46 and the liquid-impermeable backsheet 25. This construction is also the simplest to make, as it does not require making changes to existing absorbent core making process, the lower ADL being additionally interposed between the absorbent core and the backsheet.

Alternatively, it is also considered that the lower ADL 56' may in some cases be used as the bottom core wrap layer 46 and be in direct contact with the layer of SAP 60 (so that there is no separate bottom core wrap layer 46). In this case, the top core wrap layer 45 and the lower ADL 56' may partly or fully enclose the SAP layer 60. This embodiment is represented in Fig. 3B for an alternative absorbent article 20'. In this construction, the core wrap is formed by the top core wrap layer 45 and the lower ADL 56'. However, such construction has drawbacks, as it requires the lower ADL 56' to be larger and longer than necessary to cover the entire layer of absorbent material 28, which has additional material costs. The basis weight and thus the material cost of the lower ADL is typically a multiple of the low basis weight SMS nonwoven than can be used as a bottom core wrap layer 46. Thus, it may be preferred that the absorbent article comprises a bottom core wrap layer 46 and a separate lower ADL 56, as illustrated in Fig. 3A and Fig. 3C.

In another alternative, as illustrated in Fig. 3C, the SAP absorbent material 60 and the lower ADL 56" may be partially or fully enclosed between the top core wrap layer 45 and bottom core wrap layer 46. The lower acquisition and distribution layer 56" is disposed in this alternative between the layer of SAP 60 and the bottom core wrap layer 46 and thus is integrated in the absorbent core. This embodiment however adds complexity to the core making process, and it is not excluded that some superabsorbent particles may penetrate the pores of the ADL impacting its fluid handling properties.

The lower ADL may be comprised of a single layer, as represented, in particular a single nonwoven layer having the required properties. Alternatively, it is not excluded that the lower ADL may be a multi-layer construction such as a laminate or an integrated layer comprising integrated sub-layers, as long as the multi-layer construction has the required properties. Such a multi-layer construction may also comprise two sub-layers each having different width, so that the lower ADL has an increased basis weight in a central longitudinally-extending region of the article, where both sub-layers are present. For the avoidance of doubt, if the absorbent article comprises a separate bottom core wrap layer 46, any such separate bottom core wrap layer having a basis weight below 20g/m$^2$ is not considered as part of the lower ADL.

The lower ADL 56 may be comprised or consists of a nonwoven layer that can serve as a temporary reservoir for liquid that has flown through the layer of SAP because it was not absorbed fast enough by the absorbent material.

Additional layers provided to an absorbent article generally increase the thickness and bulk of the article, thereby reducing wearer comfort. Also, increased bulk is generally not desirable, especially between the wearer's legs. Therefore, it may be desirable to limit the caliper of the lower ADL to be in the range of from 0.3 mm optionally up to e.g. 4 mm, measured at 0.85kPa pressure according to the Caliper Measurement Method described herein.

The basis weight of the lower ADL may typically (but not necessarily) be in the range of from 20 g/m$^2$ to 100 g/m$^2$, or from 25 g/m$^2$ to 80 g/m$^2$, or from 30 g/m$^2$ to 50 g/m$^2$. The basis weight of the lower ADL, at least when comprised of a single layer, is typically homogeneous throughout the length and width of the lower ADL (i.e. in the longitudinal and transverse direction). The basis weight of a material is typically provided by the supplier, and can also be calculated by dividing the weight of the lower ADL by its surface.

The lower ADL may have a smaller extension in the longitudinal and/or transverse direction than the layer of SAP 60, such that the layer of absorbent material extends beyond the lower ADL in longitudinal and/or transverse direction. The SAP layer 60 may also extend beyond the upper ADS in the longitudinal and/or transverse direction.

Alternatively, the lower ADL may have a larger extension in the longitudinal and/or transverse direction than the layer of absorbent material, such that the lower ADL extends beyond the layer of absorbent material in the longitudinal and/or transverse direction. This may be desirable when the layer of absorbent material is in direct contact with the lower ADL (i.e. when there is no lower substrate layer between the layer of absorbent material and the lower ADL), as illustrated in Fig. 3B. In such configurations, the layer of SAP may be deposited on either the top core wrap layer 46 or the lower ADL 56, or partially on each layer.

The lower ADL is preferably free of superabsorbent polymers. The lower ADL may comprise or consist of a nonwoven layer. The nonwoven layer may be any type of conventional nonwovens and fibers, as long as the properties required are met. Carded nonwovens (made of staple fibers) were found particularly suitable. Carded nonwovens may be calendar bonded or air-through bonded, as is known in the art. The nonwoven layer may also be a spunbond or meltblown nonwoven web (made of continuous fibers) or a nonwoven with spunbond and meltblown layers (e.g. an SMS, SMMS, SMSS or the like).

Air-through bonded nonwoven generally have high loft. Hence, they have a porous structure to provide void volume for absorbing and temporarily holding liquid. At the same time, they provide softness and do not have an excessively high bending stiffness.

The lower ADL may comprise at least 30 weight %, optionally at last 50% and up to 100 weight % of crimped fibers based on the total weight of the lower acquisition and distribution layer. The crimped fibers may have two-dimensional crimp, three dimensional crimp or a combination of two- and three-dimensional crimp. Typically, in the carded process, all or most fibers are two-dimensionally crimped (zigzag), whereas eccentric bicomponent fibers may be typically three-dimensional crimped. Crimped fibers may help driving the bulkiness and void volume of the nonwoven.

The ADL layer, and in particular a nonwoven layer thereof, may be made or comprise of synthetic fibers. Particularly suitable synthetic fibers are made of polyolefins (e.g. polyethylene, polypropylene or mixtures or combinations thereof), polyethylene terephthalate (PET), co-PET, polylactic acid (PLA), polyhydroxy alkanoid (PHA), or combinations or mixtures thereof. The fibers may be continuous or staple fibers.

The fibers may be monocomponent fibers or multicomponent fibers, such as bicomponent fibers. If the fibers comprised by the lower ADL are bicomponent fibers, they have a core-sheath configuration, wherein the core component has a higher melting point than the sheath component.

The fibers comprised by the lower ADL are preferably staple fibers. Similar to a nonwoven web made of continuous fibers, a nonwoven web of staple fibers is preferably air-through bonded. In addition to hydroentanglement (spunlace) or air-through bonding, the nonwoven web of staple fibers may or may not have undergone some localized bonding with heat and/or pressure (e.g. point bonding/calendar bonding), introducing localized bond regions where the fibers are fused to each other.

Irrespective whether the nonwoven web is made of continuous fibers or staple fibers, the localized bonding should however not bond an excessively large surface area, thus negatively impacting the loft and void volume of the nonwoven web. Preferably, the total bond area obtained by localized bonding with heat and/or pressure (in addition to hydroentanglement or air-through bonding) should not be more than 20%, or not be more than 15%, or not be more than 10% of the total surface area of the nonwoven web.

Through-air bonding (interchangeably used with the term "air-through bonding") means a process of bonding staple fibers or continuous fibers by forcing air through the nonwoven web, wherein the air is sufficiently hot to melt (or at least partly melt, or melt to a state where the fiber surface becomes sufficiently tacky) the polymer of a fiber or, if the fibers are multicomponent fibers, wherein the air is sufficiently hot to melt (or at least partly melt, or melt to a state where the fiber surface becomes sufficiently tacky) one of the polymers of which the fibers of the nonwoven web are made. The air velocity is typically between 30 and 90 meter per minute and the dwell time may be as long as 6 seconds. The melting and re-solidification of the polymer provide the bonding between different fibers.

The hot air melts the staple or continuous fiber, or, for multicomponent fibers, the lower melting polymer component of the fiber and thereby forms bonds between the staple fibers to consolidate and integrate the layer of staple fibers into a web.

The lower ADL may comprise multicomponent fibers at least partially forming the nonwoven. The fibers of the nonwoven comprised by the lower ADL, may comprise at least 30 weight-%, or at least 40 weight-%, or at least 50 weight-%, or at least 70 weight-%, or at least 90 weight-% or 100 weight-% of multicomponent fibers based on the total weight of the nonwoven comprised by the lower acquisition and distribution layer. The multicomponent fibers may be bicomponent fibers, such as core-sheath or side-by-side bicomponent fibers.

Alternatively, the nonwoven layer comprised by or forming the lower ADL may comprise monocomponent fibers. The fibers of the nonwoven comprised by the lower acquisition and distribution layer, may comprise at least 30 weight-%, or at least 40 weight-%, or at least 50 weight-%, or at least 70 weight-%, or at least 90 weight-% or 100 weight-% of monocomponent fibers based on the total weight of the nonwoven comprised by the lower acquisition and distribution layer. The nonwoven web comprised by or forming the lower ADL may comprise a mixture of monocomponent fiber and multicomponent fibers.

In general, the fiber dtex (linked to the fiber's diameter) is directly impacting the pore size of the material and therefore especially the capillary pressure and permeability/strike-in and strike-through of the material. At a given basis weight, the lower the dtex, the lower the permeability and higher the capillary pressure. The lower acquisition and distribution layer may comprise fibers having at least 50%, or at least 70%, or at least 80% and up to 100% by weight of fibers having a denier below 10 dtex.

The lower ADL may advantageously comprise a hydrophilic agent, especially if the lower ADL comprise or consists of synthetic fibers that are inherently hydrophobic. Any conventional hydrophilic treatments may be used to provide the hydrophilic agent. Typically, a web such as a nonwoven can be externally coated by a surfactant directly or via an oil/emulsion. Alternatively, hydrophilic melt additives can be added in the polymer melt used to make the fibers, as is known in the art. Hydrophilic melt additives are amphiphilic molecules having a hydrophilic head and a hydrophobic tail. The hydrophilic head is oriented towards the surface of the adhesive, thus providing for the hydrophilic character of the adhesive, while the hydrophobic head remains in the polymer matrix.

Hydrophilic melt additives are typically compounded in a masterbatch in the form of pellets than can be incorporated by homogenous mixing in the molten polyolefin. Commercial examples of hotmelt additives particularly compatible with a propylene-based metallocene-catalyzed polyolefin are PPM 15560 from Techmer (hydrophilic PP masterbatch) and Brij S2 (Croda). Further, in order of declining preference, Brij S10 (from Croda,) Unithox 450, Unithox 720 and Unithox 750 (from Baker Hughes) can be used. PPM 15560 is preferably used in a dosage of 0.5 weight percent of the masterbatch, Brij S2 and Brij S10 in a dosage of preferably 2 weight percent of the active. Techsurf® melt additives from Techmer have been used to impart hydrophilicity to polyolefin fibers, nonwoven fabrics, and specialty plastic applications, and are useful in the present invention.

US6,146,757 discloses a hydrophilic melt additive comprising a blend of a first wetting agent and a second wetting agent. The first wetting agent is at least one water insoluble nonionic alkoxylated alkyl phenol, and the second wetting agent is at least one compound selected from the group consisting of an alkoxylated fatty alcohol and a watersoluble, nonionic, nonhydrolyzable polyoxyalkylene-modified organosilicone polymer.

The lower ADL 56 and bottom core wrap layer 46 may advantageously be both hydrophilic. The lower ADL may be optionally less hydrophilic than the bottom core wrap layer.

Packages

A plurality of absorbent articles according to the invention may be packaged in a package for transport and sale. At least 50% of the articles, and preferably all the articles, in the package may be according to the invention. The articles may be folded and packaged as is known in the art. The package may be for example a plastic bag or a cardboard box. Diapers may typically bi-folded along the transversal axis and the ears folded inwardly before being packaged. The absorbent articles may be packed under compression so as to reduce the size of the packages, while still providing an adequate number of absorbent articles per package. By packaging the absorbent articles under compression, caregivers can easily handle and store the packages, while also providing distribution and inventory savings to manufacturers owing to the size of the packages.

The absorbent articles may thus be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height" or "IBSH") divided by the height of a stack of 10 folded articles of the same type before compression, multiplied by 100; i.e. (1-IBSH/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression are sampled from the production line between the folding unit and the stack packing unit. The stack height before compression is measured by taking 10 articles before compression and packing, and measuring their stack height as indicated for the IBSH.

Packages of the absorbent articles of the present disclosure may in particular have an In-Bag Stack Height of less than 110 mm, less than 105 mm, less than 100 mm, less than 95 mm, less than 90 mm, specifically reciting all 0.1 mm increments within the specified ranges and all ranges formed therein or thereby (the In-Bag Stack Height Test is described in details in US2014/0143180A1). For each of the values indicated in the previous sentence, it may be desirable to have an In-Bag Stack Height of greater than 60, or greater than 70 mm, or greater than 75 mm, or greater than 80 mm. Alternatively, packages of the absorbent articles of the present disclosure may have an In-Bag Stack Height of from 60 mm to 110 mm, from 65 mm to 110 mm, from 70 mm to 110 mm, from 75 mm to 105 mm, or from 80 mm to 100 mm, specifically reciting all 0.1 mm increments within the specified ranges and all ranges formed therein or thereby.

Bio-sourced materials

Components of the disposable absorbent article (i.e., diaper, pant, sanitary napkin, pantiliner, etc.) of the present invention can at least partially be comprised of bio-sourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0152812A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, barrier leg cuff nonwovens, superabsorbent material, upper and lower core wrap layer, adhesives, fastener hooks, and fastener landing zone nonwovens and film based. For example, the upper and/or lower acquisition and distribution layer of the present invention may at least partially be comprised of bio-sourced content.

The disposable absorbent article component may comprise a bio-based content value from about 10% to about 100% using ASTM D6866-10, method B, in another embodiment, from about 25% to about 75%, and in yet another embodiment, from about 50% to about 60% using ASTM D6866-10, method B.

In order to apply the methodology of ASTM D6866-10 to determine the bio-based content of any disposable absorbent article component, a representative sample of the disposable absorbent article component must be obtained for testing. Thereto, the disposable absorbent article component may be ground into particulates less than about 20 mesh using known grinding methods (e.g., Wiley® mill), and a representative sample of suitable mass taken from the randomly mixed particles.

Examples and Data

SAP Materials:

Table 1 below displays the properties of different SAPs that were used to make diaper examples. Comparative SAP1 ("COMP SAP1") and Comparative SAP2 ("COMP SAP2") were both Aqualic CA L825, from Nippon Shokubai Co., Ltd. in Himeji, but taken from different packaging. The properties were measured as indicated further below in the "Test Methods" Section.

Table 1

| Material | UPM [UPM units] | EFFC [g/g] | SAP CRC [g/g] | AAP [g/g] | T20 [s] |
|---|---|---|---|---|---|
| COMP SAP1 | 34 | 26.1 | 27.5 | 24.7 | 109 |
| COMP SAP2 | 37 | 26.6 | 27.8 | 25.4 | 118 |
| SAP1 | 54 | 25.1 | 26.4 | 23.9 | 118 |
| SAP2 | 63 | 24.5 | 25.6 | 23.3 | 107 |
| SAP3 | 111 | 22.6 | 23.7 | 21.6 | 126 |
| SAP4 | 66 | 25.0 | 26.5 | 23.6 | 132 |
| SAP5 | 49 | 25.4 | 26.8 | 24.0 | 121 |

SAP1-5 according to the invention were obtained by surface cross-linking of either COMP SAP1 or COMP SAP2 to increase their Urine Permeability Measurement ("UPM") values. As can be seen from this Table 1, surface cross-linking can largely increase the UPM values while moderately decreasing the EFFC. A detailed description of the cross-linking method is further described in the section "SAP1-5 Method of Making" below.

Examples of absorbent articles with lower ADL

COMP SAP1, SAP1 and SAP2 were used to hand-make absorbent articles in the form of diaper comprising an airfelt-free core (as commercially used in Pamper® diapers). The diapers all had a 30 gsm air-through bonded (ATB) acquisition layer, and a 50 gsm Spunlace (SL) as distribution layer. A lower ADL (carded air-through bonded "C-TAB") was also placed between the absorbent core and the backsheet. The only difference between the four examples of diapers tested were the nature and amount of the SAP used in the absorbent core, as indicated below in Table 2.

The diapers thus obtained were tested according to the C-SABAP method (Curved-Speed of Acquisition with Balloon Applied Pressure). C-SABAP determines the time required to absorb a predetermined amount of saline solution while the diaper is held in a slightly curved position and placed on a latex film which is inflated by pressurized air at 2.07 kPa (0.30 psi) and monitored by a digital manometer. The speed of acquisition was measured on 4 replicates for each type of diapers. Four gushes of each 75 ml of colored saline solution (0.9 w.%) were applied sequentially at a rate 15 ml/s, with 5 minutes between each gush. The acquisition time for each gush is recorded from the time the fluid application starts to the time when fluid is not present in the surface of the TS over the application area. The liquid is delivered in the diaper at 102 mm from the front of the absorbent core, and centered in transverse direction. Lower number of acquisition time are desired and indicate a faster absorption speed of the absorbent article.

The acquisition time in s for these four consecutive gushes of saline was measured for 8 diapers per leg and the average results indicated in Table 2 below, with the standard deviation in brackets.

Table 2

| Example # | Ex. 1 (comparative) | Ex. 2 | Ex. 3 | Ex. 4 |
|---|---|---|---|---|
| SAP Type | COMP SAP1 | SAP2 | SAP2 | SAP1 |
| UPM [$10^{-7}$ $(cm^3.s)/g$] | 34 | 63 | 63 | 54 |
| EFFC [g/g] | 26.1 | 24.5 | 24.5 | 25.1 |
| SAP amount [g] | 12.6 | 12.6 | 13.5 | 12.6 |
| Acquisition layer | 30 gsm ATB | 30 gsm ATB | 30 gsm ATB | 30 gsm ATB |
| Distribution Layer | 50 gsm SL | 50 gsm SL | 50 gsm SL | 50 gsm SL |
| Lower ADL | 40 gsm C-TAB | 40 gsm C-TAB | 40 gsm C-TAB | 40 gsm C-TAB |
| Total Core EFFC [g] | 329.3 | 308.1 | 330.2 | 316.7 |
| Total Core CRC [g] | 346.9 | 323.1 | 346.2 | 332.3 |
| Total Core AAP [g] | 311.7 | 293.2 | 314.1 | 301.1 |
| 1st Acq time [s] | 78 (2) | 65 (2) | 64 (2) | 70 (2) |
| 2nd Acq time [s] | 226 (9) | 230 (11) | 219 (8) | 238 (5) |
| 3rd Acq time [s] | 392 (14) | 420 (14) | 378 (19) | 437 (38) |
| 4th Acq time [s] | 649 (18) | 683 (21) | 628 (18) | 753 (25) |

As can be seen in Table 2, the inventive diapers (examples 2 - 4) have a lower first acquisition time with the SAP having a UPM value above 50 UPM units. Examples 2 and 3 further offered parity performance for 2nd, 3rd and 4th gush even as they a lower or equivalent total core EFFC value vs. Comparative Example 1. Example 3 has the same Total EFFC as Comparative example 1, but shows lower acquisition time for each of the 4 gushes.

The data shows that at equivalent overall core capacity (Total Core EFFC), higher permeability SAP are more effective at reducing acquisition time. Even for a lower Total Core EFFC, acquisition speed could be at least partially improved relative to the comparative SAP. While not desired to be bound by theory, it is believed that the right balance of capacity and permeability can provide the desired performance for an airfelt-free diaper comprising an upper acquisition system without unbonded cross-linked cellulose fibers.

Absorbent articles without lower ADL

Further test diapers were hand-made as indicated in Table 3 below using COMP SAP2, SAP3, SAP4 and SAP5 and were tested as indicated previously. The acquisition layer was a 43 gsm resin bonded (RB), carded nonwoven. The distribution layer was a 75 gsm spunlace with 40% 1.3 dtex Viscose, 30% 4.4 dtex PET/CoPET and 30% 10 dtex PET HS. The diapers in this test series did not have a lower acquisition and distribution layer.

Table 3

| Example | Ex. 5 (Comparative) | Ex. 6 | Ex. 7 | Ex. 8 | Ex. 9 |
|---|---|---|---|---|---|
| SAP Type | COMP SAP2 | SAP4 | SAP5 | SAP3 | SAP3 |
| UPM | 37 | 66 | 49 | 111 | 111 |
| SAP amount [g] | 15.5 | 15.2 | 15 | 15.2 | 16.8 |
| Acquisition layer | 43 gsm RB | 43 gsm RB | 43 gsm RB | 43 gsm RB | 43 gsm RB |

(continued)

| Example | Ex. 5 (Comparative) | Ex. 6 | Ex. 7 | Ex. 8 | Ex. 9 |
|---|---|---|---|---|---|
| Distribution Layer | 75 gsm SL | 75 gsm SL | 75 gsm SL | 75 gsm SL | 75 gsm SL |
| Lower ADL | None | None | None | None | None |
| Total Core EFFC [g] | 412.3 | 380.0 | 381.0 | 343.5 | 379.7 |
| Total Core CRC [g] | 430.9 | 402.8 | 402.0 | 360.2 | 398.2 |
| Total Core AAP [g] | 393.7 | 358.7 | 360.0 | 328.3 | 362.9 |
| 1st Acq time [s] | 56 (2) | 56 (1) | 53 (2) | 50 (2) | 46 (1) |
| 2nd Acq time [s] | 107 (4) | 111 (5) | 107 (6) | 106 (5) | 95 (4) |
| 3rd Acq time [s] | 174 (12) | 180 (9) | 184 (9) | 187 (11) | 156 (7) |
| 4th Acq time [s] | 316 (24) | 313 (21) | 335 (28) | 335 (28) | 301 (28) |

Inventive examples 6 and 7 show that using SAP having a higher UPM provide comparable acquisition speed for the four gushes of the test, even when the Total Core EFFC is significantly lower than for comparative example 5.

Example 8 uses SAP3, which has a very high UPM value, but the acquisition speed recorded is not significantly different from the previous examples 6 and 7, or even slightly lower than for the 4th acquisition time. Example 9 shows that performance could be significantly increased, but at the cost of 1.6 g of additional SAP3. While not wishing to be bound by theory, it is believed that for a given SAP, the EFFC and UPM are generally inversely correlated. Thus, while SAP3 has a very high permeability, its EFFC is significantly lower than for example SAP4 or SAP5 resulting in overall lower core capacity at equal SAP amount. Lower core capacity may be an issue for load situation.

Example 6 provided the best balance between decreased capacity and increased permeability, while using 0.3g less SAP than example 5. It is believed that a performance sweet spot is found at about 50 to about 100 UPM units, preferably at about 55 to about 90 UPM units, where it is possible to decrease the superabsorbent amount without affecting the performance of the absorbent core.

SAP1-5 Method of making

The inventive examples SAP1-SAP5 were obtained by surface crosslinking treatment with Ethylene Carbonate (EC) of COMP SAP1 and COMP SAP 2 (surface crosslinker application in fluidized bed) as detailed below.

Lab conditions:

Ambient conditions of 23±2°C and relative humidity of 45±10%.

Surface Crosslinking Chemicals: EC: Ethylene Carbonate ("EC") anhydrous, 99% purity / Sigma-Aldrich.

Equipment:

ProCell Labsystem Pro by Glatt Ingenieurtechnik GmbH with the Coater Module GF3 (reactor [B206010] with process insert [B203010]) with the transitional housing [B203000] and Wurster insert (70mm diameter and 190mm height)) with Wurster bottom "Type B" and cyclone [F121490]; or similar equipment.

The spray nozzle was a two-stream bottom spray nozzle (Schlick two-stream nozzle, model # 970 form 0S4). Nozzle cap position is adjusted to flush with the tip of the nozzle pipe. Project number: W51505 in 2013.

The system is run without feedback stream of fines from the cyclone.

Pump: Ismatec pump ISM 404B, with pump head ISM 720A.

Hose: silicone (peroxid-cured) ID=2.06 mm

Preparation of 300 g Surface Crosslinker solution:

The original bottle of EC was placed into the circulation oven @60°C for 2 hours to reach liquid form. The amount of 299.93 g of deionized water was put into a beaker with magnetic stirrer. The amount of 0.057 cm$^3$ (equivalent to 0.080 g) of EC was taken from the bottle with a pipette with combi tips advanced and transferred into the beaker.

The solution was stirred at room temperature for 5 minutes.

264 g was taken from the prepared solution, transferred to another beaker, and used for coating. The beaker with the aqueous EC solution is placed on a balance during the coating operation to control the spray rate of the coating.

Equipment Preparation:

Before the coating was started, the equipment was closed, started and the pressured air valve is opened. The equipment was preheated for about 30 min with air flow of 65 $Nm^3$/h at 70°C set point for fluidization air.
Pump calibration: The peristaltic pump with the silicon hose is calibrated with about 20 g of deionized water to a flow rate of 2.5 g/min $\pm$ 0.1 g/min).

Coating:

600.0 g $\pm$ 1.0 g of the starting SAP (COMP SAP1) were placed in the GF3 process vessel.
The equipment was closed, and the equipment was started in the following order at the respective settings:

1) The fan was started, setting 65 $Nm^3$/h, product temperature 70°C.
2) The nozzle air was started at 1.2 bar spray pressure.
3) The heating was started (product temperature set point 70°C).
4) When the temperature inside the coating vessel reached about 70°C, the liquid port of the spray nozzle was connected via the hose mounted in the pump head to the Ethylene carbonate solution and the pump was started. The solution was sprayed at a spray rate of about 2.5 g/min $\pm$ 0.1 g/min onto the Comp AGM 1 in the reactor. For the duration of the experiment, the product temperature was controlled within the range from 68°C to 72°C and the fluidization air flow rate within 60 and 70 $m^3$/h. The spray rate of the coating agent, the fluidization air temperature or product temperature, respectively, and the fluidization air flowrate was set such that the water-absorbing polymer particles were not getting sticky and no additional drying is needed after the coating is completed.

In total, 264.0 g $\pm$ 0.2 g of aqueous EC solution was sprayed onto the Comp AGM 1 during coating. After that, the equipment was stopped as following:

1) The heater was stopped.
2) The fan was stopped.
3) The spray air of the nozzle was stopped.
4) The coated water-absorbing polymer particles were discharged from the reactor vessel into a stainless steel bowl and weighed to the nearest 0.1 g. The material in the expansion room above the reactor vessel and the material in the sealing area between reactor vessel and expansion room is not collected, but discarded. In case the weight of collected coated water-absorbing polymer particles in the stainless steel bowl deviates more than 15 w% from the in-weight of Comp AGM (here 600.0 g $\pm$ 1.0 g) the material is discarded, and the experiment needs to be repeated.

Heating / Curing:

The coated water-absorbing polymer particles were evenly distributed onto two Teflon coated baking trays (41 x 31 x 10 cm). The baking trays were covered with an aluminum foil and transferred into a circulation oven preheated to 192°C. The temperature in the oven was controlled within the range from 191 to 193°C. The coated water-absorbing polymer particles stay inside the oven for the curing time as listed in table 3 below. After that, the coated water-absorbing polymer particles were removed from the oven, remaining in the aluminum foil covered dish, and were let cooled down to room temperature.
After the coated water-absorbing polymer particles were cooled to room temperature, they were sieved via sieves of about 20 cm in diameter. A stack of sieves with the following mesh sizes (sequence from top to bottom) is used: 710 $\mu$m, 45 $\mu$m and collecting pan. The superabsorbent particles sample were loaded to the top sieve (i.e. 710 $\mu$m) and sieved via a sieve machine (e.g. "AS 400 control available from Retsch GmbH, Haan, Germany) for 3 min at 1 mm/g.
The fraction of coated water-absorbing polymer particles of the size from 45 $\mu$m to 710 $\mu$m represents the respective SAP samples as listed in table 1.
After sieving, SAP pre2 was heat treated / cured another time in a circulation oven. The oven has been preheated to 192°C. The temperature in the oven is controlled within the range from 191 to 193°C. The resulting sample is SAP2, with total heat treatment / curing time of 102 min.

Table 4: Curing Times

| AGM | Curing Duration at 192°C | Yield [g] |
|---|---|---|
| SAP1 | 60 min | 566 |
| SAP pre2 | 42 min | 553 |
| SAP2 | Test AGM pre2 + 60 min | as SAP pre2 |
| Note: The desired UPM level can easily be adjusted via longer or shorter duration of the heat treatment / curing, with longer heat treatment / curing duration leading to higher UPM, i.e. to higher SAP permeability. | | |

SAP3-5 were obtained like SAP1-2, but starting from COMP SAP 2 and using the following curing conditions.

Table 5: Curing Times

| AGM | Curing Duration at 192°C | Yield [g] |
|---|---|---|
| SAP3 | 107 | 555 |
| SAP4 | 60 | 560 |
| SAP5 | 45 | 562 |

TEST METHODS

Centrifuge Retention Capacity (CRC) test method

The CRC measures the liquid absorbed by the superabsorbent polymer particles for free swelling in excess saline solution. The CRC is measured according to EDANA method NWSP 241.0.R2(19).

Absorption Against Pressure (AAP) test method

The AAP is measured according to EDANA method NWSP 242.0.R2 (19) with applied pressure of 0.7 psi.

Effective Capacity (EFFC)

The Effective Capacity represents an average of the value of Centrifuge Retention Capacity (CRC) and of the value of Absorption Against Pressure (AAP) of the superabsorbent polymer particles.
The Effective Capacity (EFFC) is calculated via the formula below: EFFC = (CRC+AAP)/2.

Bulk density test method

The bulk density test method refers to the EDANA method NWSP 251.0.R2 (19).

Urine Permeability Measurement (UPM) Method

Lab Conditions:

This test has to be performed in a climate conditioned room at standard conditions of 23°C $\pm$ 2°C temperature and 45% $\pm$ 10% relative humidity.

Urine Permeability Measurement System

This method determined the permeability of a swollen hydrogel layer 1318. The equipment used for this method is described below.

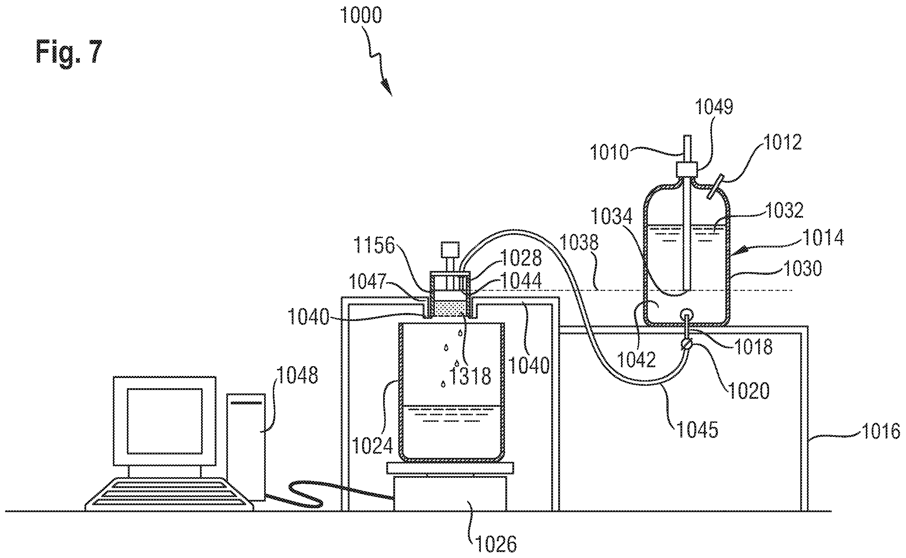

Fig. 7 shows permeability measurement system 1000 set-up with the constant hydrostatic head reservoir 1014, open-ended tube for air admittance 1010, stoppered vent for refilling 1012, laboratory rack 1016, delivery tube 1018 with flexible tube 1045 with Tygon tube nozzle 1044, stopcock 1020, cover plate 1047 and supporting ring 1040, receiving vessel 1024, balance 1026 and piston/cylinder assembly 1028.

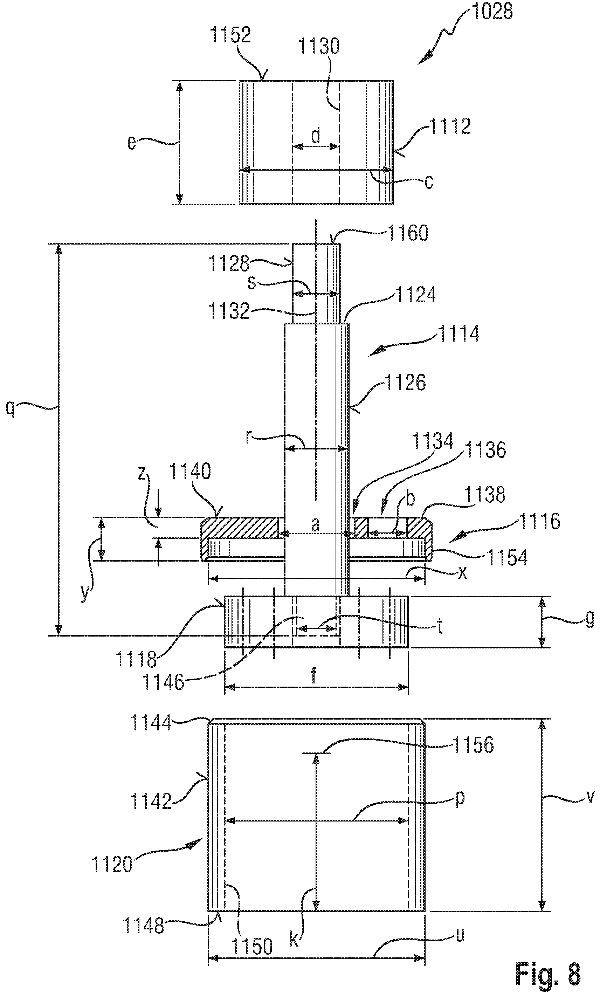

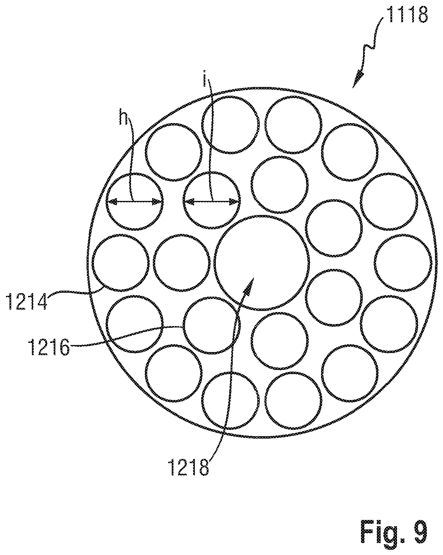

Fig. 8 shows the piston/cylinder assembly 1028 comprising a metal weight 1112, piston shaft 1114, piston head 1118, lid 1116, and cylinder 1120. The cylinder 1120 is made of transparent polycarbonate (e.g., Lexan®) and has an inner diameter p of 6.00 cm (area = 28.27 cm²) with inner cylinder walls 1150 which are smooth. The bottom 1148 of the cylinder 1120 is faced with a stainless-steel screen cloth (ISO 9044 Material 1.4401, mesh size 0.038 mm, wire diameter 0.025 mm) (not shown) that is bi-axially stretched to tautness prior to attachment to the bottom 1148 of the cylinder 1120. The piston shaft 1114 is made of transparent polycarbonate (e.g., Lexan®) and has an overall length q of approximately 127 mm. A middle portion 1126 of the piston shaft 1114 has a diameter r of 22.15 (± 0.02) mm. An upper portion 1128 of the piston shaft 1114 has a diameter s of 15.8 mm, forming a shoulder 1124. A lower portion 1146 of the piston shaft 1114 has a diameter t of approximately 5/8 inch (15.9 mm) and is threaded to screw firmly into the center hole 1218 (see Fig. 9) of the piston head 1118. The piston head 1118 is perforated, made of transparent polycarbonate (e.g., Lexan®), and is also screened with a stretched stainless-steel screen cloth (ISO 9044 Material 1.4401, mesh size 0.038 mm, wire diameter 0.025 mm) (not shown). The weight 1112 is stainless steel, has a center bore 1130, slides onto the upper portion 1128 of piston shaft 1114 and rests on the shoulder 1124. The combined weight of the piston head 1118, piston shaft 1114 and weight 1112 is 596 g (± 6 g), which corresponds to 0.30 psi over the inner area of the cylinder 1120. The combined weight may be adjusted by drilling a blind hole down a central axis 1132 of the piston shaft 1114 to remove material and/or provide a cavity to add weight. The cylinder lid 1116 has a first lid opening 1134 in its center for vertically aligning the piston shaft 1114 and a second lid opening 1136 near the edge 1138 for introducing fluid from the constant hydrostatic head reservoir 1014 into the cylinder 1120.

A first linear index mark (not shown) is scribed radially along the upper surface 1152 of the weight 1112, the first linear index mark being transverse to the central axis 1132 of the piston shaft 1114. A corresponding second linear index mark (not shown) is scribed radially along the top surface 1160 of the piston shaft 1114, the second linear index mark being transverse to the central axis 1132 of the piston shaft 1114. A corresponding third linear index mark (not shown) is scribed along the middle portion 1126 of the piston shaft 1114, the third linear index mark being parallel with the central axis 1132 of the piston shaft 1114. A corresponding fourth linear index mark (not shown) is scribed radially along the upper surface 1140 of the cylinder lid 1116, the fourth linear index mark being transverse to the central axis 1132 of the piston shaft 1114. Further, a corresponding fifth linear index mark (not shown) is scribed along a lip 1154 of the cylinder lid 1116, the fifth linear index mark being parallel with the central axis 1132 of the piston shaft 1114. A corresponding sixth linear index mark (not shown) is scribed along the outer cylinder wall 1142, the sixth linear index mark being parallel with the central axis 1132 of the piston shaft 1114. Alignment of the first, second, third, fourth, fifth, and sixth linear index marks allows for the weight 1112, piston shaft 1114, cylinder lid 1116, and cylinder 1120 to be repositioned with the same orientation relative to one another for each measurement.

The cylinder 1120 specification details are:

Outer diameter u of the Cylinder 1120: 70.35 mm (±0.05 mm)
Inner diameter p of the Cylinder 1120: 60.0 mm (±0.05 mm)
Height v of the Cylinder 1120: 60.5 mm. Cylinder height must not be lower than 55.0 mm!

The cylinder lid 1116 specification details are:

Outer diameter w of cylinder lid 1116: 76.05 mm (±0.05 mm)
Inner diameter x of cylinder lid 1116: 70.5 mm (±0.05 mm)
Thickness y of cylinder lid 1116 including lip 1154: 12.7 mm
Thickness z of cylinder lid 1116 without lip 1154: 6.35 mm
Diameter a of first lid opening 1134: 22.25 mm (±0.02 mm)
Diameter b of second lid opening 1136: 12.7 mm (±0.1 mm)
Distance between centers of first and second lid openings 1134 and 1136: 23.5 mm

The weight 1112 specification details are:

Outer diameter c: 50.0 mm
Diameter d of center bore 1130: 16.0 mm
Height e: 39.0 mm

The piston head 1118 specification details are:

Diameter f: 59.7 mm (±0.05 mm)
Height g: 16.5 mm. Piston head height must not be less than 15.0 mm.
Outer holes 1214 (14 total) with a 9.30 (±0.25) mm diameter h, outer holes 1214 equally spaced with centers being 23.9 mm from the center of center hole 1218. Inner holes 1216 (7 total) with a 9.30 (±0.25) mm diameter i, inner holes 1216 equally spaced with centers being 13.4 mm from the center of center hole 1218.

Center hole 1218 has a diameter j of approximately 5/8 inches (15.9 mm) and is threaded to accept a lower portion 1146 of piston shaft 1114.

Prior to use, the stainless steel screens (not shown) of the piston head 1118 and cylinder 1120 should be inspected for clogging, holes or over-stretching and replaced when necessary. A urine permeability measurement apparatus with damaged screen can deliver erroneous UPM results, and must not be used until the screen has been replaced.

A 5.00 cm mark 1156 is scribed on the cylinder 1120 at a height k of 5.00 cm ($\pm$0.05 cm) above the screen (not shown) attached to the bottom 1148 of the cylinder 1120. This marks the fluid level to be maintained during the analysis. Maintenance of correct and constant fluid level (hydrostatic pressure) is critical for measurement accuracy.

A constant hydrostatic head reservoir 1014 is used to deliver salt solution 1032 to the cylinder 1120 and to maintain the level of salt solution 1032 at a height k of 5.00 cm above the screen (not shown) attached to the bottom 1148 of the cylinder 1120. The bottom 1034 of the air-intake tube 1010 is positioned so as to maintain the salt solution 1032 level in the cylinder 1120 at the required 5.00 cm height k during the measurement, i.e., bottom 1034 of the air tube 1010 is in approximately same plane 1038 as the 5.00 cm mark 1156 on the cylinder 1120 as it sits on the cover plate 1047 and supporting ring 1040 (with circular inner opening of not less than 64 mm diameter) above the receiving vessel 1024.

The cover plate 1047 and supporting ring 1040 are parts as used in the equipment used for the method "K(t) Test Method (Dynamic Effective Permeability and Uptake Kinetics Measurement Test method)" as described e.g. in WO2021/188330 and is called "Zeitabhängiger Durchlässigkeitsprüfstand" or "Time Dependent Permeability Tester", Equipment No. 03-080578 and is commercially available at BRAUN GmbH, Frankfurter Str. 145, 61476 Kronberg, Germany. Upon request, detailed technical drawings are also available.

Proper height alignment of the air-intake tube 1010 and the 5.00 cm mark 1156 on the cylinder 1120 is critical to the analysis. A suitable reservoir 1014 consists of a jar 1030 containing: a horizontally oriented L-shaped delivery tube 1018 connected to a flexible tube 1045 (e.g. Tygon tube, capable to connect nozzle and reservoir outlet) and to a Tygon tube nozzle 1044 (inner diameter at least 6.0 mm, length appr. 5.0 cm) for fluid delivery, a vertically oriented open-ended tube 1010 for admitting air at a fixed height within the constant hydrostatic head reservoir 1014, and a stoppered vent 1012 for refilling the constant hydrostatic head reservoir 1014. Tube 1010 has an internal diameter of approximately 12 mm, but not less than 10.5 mm. The delivery tube 1018, positioned near the bottom 1042 of the constant hydrostatic head reservoir 1014, contains a stopcock 1020 for starting/stopping the delivery of salt solution 1032. The outlet 1044 of the delivery flexible tube 1045 is dimensioned (e.g. outer diameter 10 mm) to be inserted through the second lid opening 1136 in the cylinder lid 1116, with its end positioned below the surface of the salt solution 1032 in the cylinder 1120 (after the 5.00 cm height of the salt solution 1032 is attained in the cylinder 1120). The air-intake tube 1010 is held in place with an O-ring collar 1049. The constant hydrostatic head reservoir 1014 can be positioned on a laboratory reck 1016 at a suitable height relative to that of the cylinder 1120. The components of the constant hydrostatic head reservoir 1014 are sized so as to rapidly fill the cylinder 1120 to the required height (i.e., hydrostatic head) and maintain this height for the duration of the measurement. The constant hydrostatic head reservoir 1014 must be capable of delivering salt solution 1032 at a flow rate of at least 2.6 g/sec for at least 10 minutes.

The piston/cylinder assembly 1028 is positioned on the supporting ring 1040 in the cover plate 1047 or suitable alternative rigid stand. The salt solution 1032 passing through the piston/cylinder assembly 1028 containing the swollen hydrogel layer 1318 is collected in a receiving vessel 1024, positioned below (but not in contact with) the piston/cylinder assembly 1028.

The receiving vessel 1024 is positioned on the balance 1026 which is accurate to at least 0.001 g. The digital output of the balance 1026 is connected to a computerized data acquisition system 1048.

*Preparation of Reagents (not illustrated)*

Jayco Synthetic Urine (JSU) 1312 is used for a swelling phase (see UPM Procedure below) and 0.118 M Sodium Chloride (NaCl) Solution 1032 is used for a flow phase (see UPM Procedure below). The following preparations are referred to a standard 1 liter volume. For preparation of volumes other than 1 liter, all quantities are scaled accordingly.

JSU: A 1L volumetric flask is filled with distilled water to 80% of its volume, and a magnetic stir bar is placed in the flask. Separately, using a weighing paper or beaker the following amounts of dry ingredients are weighed to within $\pm$ 0.01 g using an analytical balance and are added quantitatively to the volumetric flask in the same order as listed below. The solution is stirred on a suitable stir plate until all the solids are dissolved, the stir bar is removed, and the solution diluted to 1L volume with distilled water. A stir bar is again inserted, and the solution stirred on a stirring plate for a few minutes more.

Quantities of salts to make 1 liter of Jayco Synthetic Urine:

Potassium Chloride (KCl) 2.00 g

Sodium Sulfate (Na2SO4) 2.00 g
Ammonium dihydrogen phosphate (NH4H2PO4) 0.85 g
Ammonium phosphate, dibasic ((NH4)2HPO4) 0.15 g
Calcium chloride (CaCl2) 0.19 g - [or hydrated calcium chloride (CaCl2•2H2O) 0.25 g]
Magnesium chloride (MgCl2) 0.23 g - [or hydrated magnesium chloride (MgCl2•6H2O) 0.50 g]

To make the preparation faster, potassium chloride, sodium sulfate, ammonium dihydrogen phosphate, ammonium phosphate (dibasic) and magnesium chloride (or hydrated magnesium chloride) are combined and dissolved in the 80% of distilled water in the 1L volumetric flask. Calcium chloride (or hydrated calcium chloride) is dissolved separately in approximately 50 ml distilled water (e.g. in a glass beaker) and the calcium chloride solution is transferred to the 1L volumetric flask after the other salts are completely dissolved therein. Afterwards, distilled water is added to 1L (1000 ml $\pm$ 0.4 ml) and the solution is stirred for a few minutes more. Jayco synthetic urine may be stored in a clean plastic container for 10 days. The solution should not be used if it becomes cloudy.

0.118 M Sodium Chloride (NaCl) Solution: 0.118 M Sodium Chloride is used as salt solution 1032. Using a weighing paper or beaker 6.90 g ($\pm$ 0.01 g) of sodium chloride is weighed and quantitatively transferred into a 1L volumetric flask (1000 ml $\pm$ 0.4 ml); and the flask is filled to volume with distilled water. A stir bar is added and the solution is mixed on a stirring plate until all the solids are dissolved.

The conductivity of the prepared Jayco solution must be in the range of appr. 7.48-7.72 mS/cm and of the prepared 0.118 M Sodium Chloride (NaCl) Solution in the range of appr. 12.34-12.66 mS/cm (e.g. measured via COND 70 INSTRUMENT without CELL, #50010522, equipped with Cell VPT51-01 C=0.1 from xs instruments or via LF 320 / Set, #300243 equipped with TetraCon 325 from WTW or COND 330i , #02420059 equipped with TetraCon 325 from WTW). The surface tension of each of the solutions must be in the range of 71-75 mN/m (e.g. measured via tensiometer K100 from Kruess with Pt plate).

*Test Preparation*

Using a solid reference cylinder weight (not shown) (50 mm diameter; 128 mm height), a caliper gauge (not shown) (measurement range 25 mm, accurate to 0.01 mm, piston pressure max. 50 g; e.g. Mitutoyo Digimatic Height Gage) is set to read zero. This operation is conveniently performed on a smooth and level bench (not shown) of at least approximately 11.5 cm x 15 cm. The piston/cylinder assembly 1028 without superabsorbent polymer particles is positioned under the caliper gauge (not shown) and a reading, L1, is recorded to the nearest 0.01 mm.

The constant hydrostatic head reservoir 1014 is filled with salt solution 1032. The bottom 1034 of the air-intake tube 1010 is positioned so as to maintain the top part (not shown) of the liquid meniscus (not shown) in the cylinder 1120 at the 5.00 cm mark 1156 during the measurement. Proper height alignment of the air-intake tube 1010 at the 5.00 cm mark 1156 on the cylinder 1120 is critical to the analysis.

The receiving vessel 1024 is placed on the balance 1026 and the digital output of the balance 1026 is connected to a computerized data acquisition system 1048. The cover plate 1047 with the supporting ring 1040 is positioned above the receiving vessel 1024.

*UPM Procedure*

1.5 g ($\pm$ 0.05g) of superabsorbent polymer particles is weighed onto a suitable weighing paper or weighing aid using an analytical balance. The moisture content of the superabsorbent polymer particles is measured according to the Edana Moisture Content Test Method NWSP 230.0.R2 (15) or via a Moisture Analyzer (HX204 from Mettler Toledo, drying temperature 130°C, starting superabsorber weight 3.0 g ($\pm$ 0.5 g), stop criterion 1 mg/ 140 s). If the moisture content of the superabsorbent polymer particles is greater than 3wt%, then the superabsorbent polymer particles are dried to a moisture level of < 3wt%, e.g. in an oven at 105°C for 3 h or e.g. at 120°C for 2 h.

The empty cylinder 1120 is placed on a level benchtop (not shown) and the superabsorbent polymer particles are quantitatively transferred into the cylinder 1120. The superabsorbent polymer particles are evenly dispersed on the screen (not shown) attached to the bottom 1148 of the cylinder 1120 while rotating the cylinder 1120, e.g. aided by a (manual or electrical) turn table (e.g. petriturn-E or petriturn-M from Schuett). It is important to have an even distribution of particles on the screen (not shown) attached to the bottom 1148 of the cylinder 1120 to obtain the highest precision result. After the superabsorbent polymer particles have been evenly distributed on the screen (not shown) attached to the bottom 1148 of the cylinder 1120 particles must not adhere to the inner cylinder walls 1150. The piston shaft 1114 is inserted through the first lid opening 1134, with the lip 1154 of the lid 1116 facing towards the piston head 1118. The piston head 1118 is carefully inserted into the cylinder 1120 to a depth of a few centimeters. The lid 1116 is then placed onto the upper rim 1144 of the cylinder 1120 while taking care to keep the piston head 1118 away from the superabsorbent polymer particles. The weight 1112 is positioned on the upper portion 1128 of the piston shaft 1114 so that it rests on the shoulder 1124 such that the first and second linear index marks are aligned. The lid 1116 and piston shaft 1126 are then carefully rotated so as to align the third, fourth, fifth, and sixth linear index marks are then aligned with the first and the second linear index marks. The piston head 1118 (via the piston shaft 1114) is then gently lowered to rest on the dry superabsorbent polymer particles. Proper seating of the lid 1116 prevents binding and assures an even distribution of the weight on the hydrogel layer 1318.

*Swelling Phase:*

A fritted disc of at least 8 cm diameter (e.g. 8-9 cm diameter) and at least 5.0 mm thickness (e.g. 5-7 mm thickness) with porosity "coarse" or "extra coarse" ( e.g. Chemglass Inc. # CG 201-51, coarse porosity; or e.g. Robu 1680 with porosity 0) 1310 is placed in a wide flat-bottomed Petri dish 1314 and JSU 1312 is added by pouring JSU 1312 onto the center of the fritted disc 1310 until JSU 1312 reaches the top surface 1316 of the fritted disc 1310. The JSU height must not exceed the height of the fritted disc 1310. It is important to avoid any air or gas bubbles entrapped in or underneath the fritted disc 1310.

The entire piston/cylinder assembly 1028 is lifted and placed on the fritted disc 1310 in the Petri dish 1314. JSU 1312 from the Petri dish 1314 passes through the fritted disc 1310 and is absorbed by the superabsorbent polymer particles (not shown) to form a hydrogel layer 1318. The JSU 1312 available in the Petri dish 1314 should be enough for all the swelling phase. If needed, more JSU 1312 may be added to the Petri dish 1314 during the hydration period to keep the JSU 1312 level at the top surface 1316 of the fritted disc 1310. After a period of 60 minutes, the piston/cylinder assembly 1028 is removed from the fritted disc 1310, taking care to ensure the hydrogel layer 1318 does not lose JSU 1312 or take in air during this procedure. The piston/cylinder assembly 1028 is placed under the caliper gauge (not shown) and a reading, L2, is recorded to the nearest 0.01 mm. If the reading changes with time, only the initial value is recorded. The thickness of the hydrogel layer 1318, L0 is determined from L2 - L1 to the nearest 0.1 mm.

The piston/cylinder assembly 1028 is transferred to the supporting ring 1040 in the cover plate 1047. The constant hydrostatic head reservoir 1014 is positioned such that the delivery tube nozzle 1044 is placed through the second lid opening 1136. The measurement is initiated in the following sequence:

a) The stopcock 1020 of the constant hydrostatic head reservoir 1014 is opened to permit the salt solution 1032 to reach the 5.00 cm mark 1156 on the cylinder 1120. This salt solution 1032 level should be obtained within 10 seconds of opening the stopcock 1020.

b) Once 5.00 cm of salt solution 1032 is attained, the data collection program is initiated.

With the aid of a computer 1048 attached to the balance 1026, the quantity g (in g to accuracy of 0.001 g) of salt solution 1032 passing through the hydrogel layer 1318 is recorded at intervals of 20 seconds for a time period of 10 minutes. At the end of 10 minutes, the stopcock 1020 on the constant hydrostatic head reservoir 1014 is closed.

The data from 60 seconds to the end of the experiment are used in the UPM calculation. The data collected prior to 60 seconds are not included in the calculation.

For each time period of 20 seconds (time $t_{(i-1)}$ to $t_i$) after the initial 60 seconds of the experiment, the respective flow rate Fs(t) (in g/s) and the respective mid-point of the time $t_{(1/2)t}$ (in s) is calculated according to the following formulas:

$$Fs_{(t)} = \frac{(g_{(i-1)} - g_{(i)})}{(t_{(i-1)} - t_{(i)})} \quad \text{and} \quad t_{(1/2)_t} = \frac{(t_{(i-1)} + t_{(i)})}{2} \quad (\text{II})$$

The flow rate Fs(t) of each time interval ($t_{(i-1)}$ to $t_i$) is plotted versus the mid-point of the time $t_{(1/2)t}$ of the time interval ($t_{(i-1)}$ to $t_i$). The intercept is calculated as Fs(t=0).

*Calculation of the Intercept:*

The intercept is calculated via a best-fit regression line, e.g. as following: the equation for the intercept of the regression line, a, is:

$$a = y_{AVG} - b \cdot x_{AVG} \quad (\text{III})$$

where the slope, b, is calculated as:

$$b = \frac{\sum (x - x_{AVG}) \cdot (y - y_{AVG})}{\sum (x - x_{AVG})^2} \quad (\text{IV})$$

and where $x_{AVG}$ and $y_{AVG}$ are the sample means AVERAGE of the known_x's and AVERAGE of the known_y's, respectively.

*Calculation of Urine Permeability Measurement Q:*

The intercept Fs(t=0) is used to calculate Q according to the following formula:

$$Q = \frac{F_s(t=0) \cdot L_0}{\rho \cdot A \cdot \Delta P} \qquad (V)$$

where the flow rate Fs(t=0) is given in g/s, $L_0$ is the initial thickness of the hydrogel layer 1318 in cm, $\rho$ is the density of the salt solution 1032 in $g/cm^3$ (e.g. 1.003 $g/cm^3$ at room temperature). A (from the equation above) is the area of the hydrogel layer 1318 in $cm^2$ (e.g. 28.27 $cm^2$), $\Delta P$ is the hydrostatic pressure in $dyne/cm^2$ (e.g. 4920 $dyne/cm^2$), and the Urine Permeability Measurement, Q, is in units of $cm^3sec/g$. The average of three determinations should be reported.

| Variable | Description | Unit |
|---|---|---|
| $g_i$ | Mass of salt solution 1032 flown through the swollen gel layer (recorded by the balance) at the time $t_i$ (accuracy 0.001 g) | g |
| $t_i$ | Time point (every 20 s) | s |
| $t_{(1/2)t}$ | Mid-point of time for the respective time interval $t_{i-1}$ to $t_i$ | s |
| $Fs_t$ | Flow Rate at the time interval $t_{i-1}$ to $t_i$ | g/s |
| Fs (t=0) | Intercept flow rate at t = 0 s from the plot of the flow rate Fs(t) vs. the mid-point of time $t_{(1/2)t}$. | g/s |
| $L_0$ | Thickness of the swollen gel layer (swollen with JSU 1312) before the salt solution 1032 flows through the gel layer. | cm |
| $\rho$ | Density of the salt solution 1032 (1.003 $g/cm^3$) | $g/cm^3$ |
| A | Area of the swollen gel layer (28.27 $cm^2$) | $cm^2$ |
| $\Delta P$ | Hydrostatic pressure across the gel layer (4920 $dyne/cm^2$) | $dyne/cm^2$ |
| Q | Urine Permeability Measurement | $cm^3$ * sec/g |

SAP K(t) Test Method

This method determines the time dependent effective permeability - SAP K(t) - and the uptake kinetics of a gel layer formed from hydrogel-forming superabsorbent polymer particles or of an absorbent structure containing such particles under a confining pressure. The objective of this method is to assess the ability of the gel layer formed from hydrogel-forming superabsorbent polymer particles or the absorbent structure containing them to acquire and distribute body fluids when the polymer is present at high concentrations in an absorbent article and exposed to mechanical pressures as they typically occur during use of the absorbent article. Darcy's law and steady-state flow methods are used to calculate effective permeability (see below). See also for example, "Absorbency," ed. By P.K. Chatterjee, Elsevier, 1982, Pages 42-43 and "Chemical Engineering Vol. II, Third Edition, J.M. Coulson and J.F. Richardson, Pergamon Press, 1978, Pages 122-127.

In contrast to previously published methods, the sample is not preswollen therefore the hydrogel is not formed by preswelling hydrogel-forming superabsorbent polymer particles in synthetic urine, but the measurement is started with a dry structure. The equipment used for this method is called 'Zeitabhängiger Durchlässigkeitsprüfstand' or 'Time Dependent Permeability Tester', Equipment No. 03-080578 and is commercially available at BRAUN GmbH, Frankfurter Str. 145, 61476 Kronberg, Germany and is described below. Upon motivated request, operating instructions, wiring diagrams and detailed technical drawings are also available.

A detailed description of the equipment (the Dynamic Effective Permeability and Uptake Kinetic Measurement System) and its handling is further disclosed in WO2015/041784A1 (Peri et al., P&G).

The average values for T20, T80%, K20, U20 and Kmin/K20 are reported from 3 replicates according to the accuracy required as known by the skilled man.

Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

**Claims**

1.  An absorbent article (20) comprising:

    - a liquid-permeable topsheet (24);
    - an absorbent core (28) comprising a top core wrap layer (45), a bottom core wrap layer (46) and a layer of superabsorbent polymer particles (60) disposed between the top core wrap layer and the bottom core wrap layer;
    - an upper acquisition-distribution system (52, 54), wherein the upper acquisition-distribution system consists of the one or more layers disposed between the topsheet and the absorbent core, wherein the upper acquisition-distribution system comprises at least one spunlace layer and wherein said upper acquisition-distribution system is free of unbonded cross-linked cellulose fibers; and
    - a liquid-impermeable backsheet (25);

    wherein the superabsorbent polymer particles have a Urine Permeability Measurement (UPM) of at least $45.10^{-7}$ $(cm^3.s)/g$, as measured by UPM Method described herein.

2.  The absorbent article of claim 1, wherein the superabsorbent polymer particles have an UPM in the range of $55.10^{-7}$ to $90.10^{-7}$ $(cm^3.s)/g$.

3.  The absorbent article of claim 1 or 2, wherein the superabsorbent polymer particles have an Effective Capacity (EFFC) above 23 g/g, wherein the Effective Capacity is measured as described herein.

4.  The absorbent article of claim 3, wherein the superabsorbent polymer particles have an EFFC in the range of from 23.5 g/g to 29 g/g.

5.  The absorbent article according to any of the preceding claims, wherein the superabsorbent polymer particles are not mixed with cellulose fibers.

6.  The absorbent article according to any of the preceding claims, wherein the upper acquisition-distribution system comprises a first layer and second layer, the first layer being closer to the topsheet and the second layer closer to the absorbent core, wherein the first layer is a nonwoven acquisition layer (52) and the second layer is a nonwoven distribution layer (54).

7.  The absorbent article according to claim 6, wherein the second layer (54) is a spunlace nonwoven.

8.  The absorbent article according to any of the preceding claims, further comprising a lower acquisition and distribution layer (56) between the layer of superabsorbent polymer particles and the backsheet, wherein the lower acquisition and distribution layer comprises or consists of a nonwoven layer, in particular a carded nonwoven layer.

9.  The absorbent article according to claim 8, wherein the lower acquisition and distribution layer has a basis weight of from 20 gsm to 100 gsm, preferably of from 30 gsm to 50 gsm.

10. The absorbent article according to any of claims 8-9, wherein the lower acquisition and distribution layer (56) comprises a surfactant coating and/or a hydrophilic melt additive.

11. The absorbent article according to any of claims 7-10, wherein the lower acquisition and distribution layer (56) is disposed between the bottom core wrap layer (46) and the backsheet (25), and wherein the lower acquisition and distribution layer (56) and the bottom core wrap layer (46) are both hydrophilic, whereas the lower acquisition and distribution layer (56) is less hydrophilic than the bottom core wrap layer (46).

12. The absorbent article according to any of the preceding claims, wherein at least some of the superabsorbent polymer particles are immobilized by a thermoplastic fibrous net on at least one of the top core wrap layer (45) or the bottom core wrap layer (46).

13. The absorbent article according to any of the preceding claims, wherein the layer of superabsorbent polymer particles (60) comprises at least one longitudinally-extending channel (26) which is free of superabsorbent polymer particles.

14. The absorbent article according to any of the preceding claims, wherein the superabsorbent polymer particles are obtained by surface cross-linking of a precursor SAP, wherein the precursor SAP is preferably internally cross-linked.

**Patentansprüche**

1. Absorptionsartikel (20), umfassend:

- eine flüssigkeitsdurchlässige Oberschicht (24);
- einen Absorptionskern (28), umfassend eine obere Kernumwicklungsschicht (45), eine untere Kernumwicklungsschicht (46) und eine Schicht aus Superabsorberpolymerteilchen (60), die zwischen der oberen Kernumwicklungsschicht und der unteren Kernumwicklungsschicht angeordnet ist;
- ein oberes Aufnahme-Verteilungssystem (52, 54), wobei das obere Aufnahme-Verteilungssystem aus der einen oder den mehreren Schichten besteht, die zwischen der Oberschicht und dem Absorptionskern angeordnet sind, wobei das obere Aufnahme-Verteilungssystem wenigstens eine Spunlacing-Schicht umfasst und wobei das obere Aufnahme-Verteilungssystem frei von ungebundenen vernetzten Zellulosefasern ist; und
- eine flüssigkeitsundurchlässige Unterschicht (25);

wobei die Superabsorberpolymerteilchen eine Urinpermeabilitätsmessung (UPM) von wenigstens $45.10^{-7}$ $(cm^3.s)/g$ aufweisen, wie durch das hierin beschriebene UPM-Verfahren gemessen.

2. Absorptionsartikel nach Anspruch 1, wobei die Superabsorberpolymerteilchen eine UPM in dem Bereich von $55.10^{-7}$ bis $90.10^{-7}$ $(cm^3.s)/g$ aufweisen.

3. Absorptionsartikel nach Anspruch 1 oder 2, wobei die Superabsorberpolymerteilchen eine effektive Kapazität (EFFC) über 23 g/g aufweisen, wobei die effektive Kapazität wie hierin beschrieben gemessen wird.

4. Absorptionsartikel nach Anspruch 3, wobei die Superabsorberpolymerteilchen eine EFFC in dem Bereich von 23,5 g/g bis 29 g/g aufweisen.

5. Absorptionsartikel nach einem der vorstehenden Ansprüche, wobei die Superabsorberpolymerteilchen nicht mit Zellulosefasern gemischt sind.

6. Absorptionsartikel nach einem der vorstehenden Ansprüche, wobei das obere Aufnahme-Verteilungssystem eine erste Schicht und eine zweite Schicht umfasst, wobei die erste Schicht näher zu der Oberschicht und die zweite Schicht näher zu dem Absorptionskern ist, wobei die erste Schicht eine Vliesaufnahmeschicht (52) ist und die zweite Schicht eine Vliesverteilungsschicht (54) ist.

7. Absorptionsartikel nach Anspruch 6, wobei die zweite Schicht (54) ein Spunlacing-Vlies ist.

8. Absorptionsartikel nach einem der vorstehenden Ansprüche, ferner umfassend eine untere Aufnahme- und Verteilungsschicht (56) zwischen der Schicht aus Superabsorberpolymerteilchen und der Unterschicht, wobei die untere Aufnahme- und Verteilungsschicht eine Vliesschicht, insbesondere eine kardierte Vliesschicht, umfasst oder daraus besteht.

9. Absorptionsartikel nach Anspruch 8, wobei die untere Aufnahme- und Verteilungsschicht ein Flächengewicht von 20 Gramm pro Quadratmeter bis 100 Gramm pro Quadratmeter, vorzugsweise von 30 Gramm pro Quadratmeter bis 50 Gramm pro Quadratmeter aufweist.

10. Absorptionsartikel nach einem der Ansprüche 8 bis 9, wobei die untere Aufnahme- und Verteilungsschicht (56) eine Tensidbeschichtung und/oder einen hydrophilen Schmelzzusatzstoff umfasst.

11. Absorptionsartikel nach einem der Ansprüche 7 bis 10, wobei die untere Aufnahme- und Verteilungsschicht (56) zwischen der unteren Kernumwicklungsschicht (46) und der Unterschicht (25) angeordnet ist, und wobei die untere Aufnahme- und Verteilungsschicht (56) und die untere Kernumwicklungsschicht (46) beide hydrophil sind, wobei die untere Aufnahme- und Verteilungsschicht (56) weniger hydrophil ist als die untere Kernumwicklungsschicht (46).

12. Absorptionsartikel nach einem der vorstehenden Ansprüche, wobei wenigstens einige der Superabsorberpolymerteilchen durch ein thermoplastisches faseriges Netz auf wenigstens einer der oberen Kernumwicklungsschicht (45) oder der unteren Kernumwicklungsschicht (46) immobilisiert sind.

13. Absorptionsartikel nach einem der vorstehenden Ansprüche, wobei die Schicht aus Superabsorberpolymerteilchen (60) wenigstens einen sich längs erstreckenden Kanal (26) umfasst, der frei von Superabsorberpolymerteilchen ist.

14. Absorptionsartikel nach einem der vorstehenden Ansprüche, wobei die Superabsorberpolymerteilchen durch Oberflächenvernetzung eines Vorläufer-SAP erhalten werden, wobei das Vorläufer-SAP vorzugsweise intern vernetzt ist.

## Revendications

1. Article absorbant (20) comprenant :

- une feuille de dessus perméable aux liquides (24) ;
- une âme absorbante (28) comprenant une couche d'enveloppe d'âme supérieure (45), une couche d'enveloppe d'âme inférieure (46) et une couche de particules de polymère superabsorbant (60) disposée entre la couche d'enveloppe d'âme supérieure et la couche d'enveloppe d'âme inférieure ;
- un système de recueil-répartition supérieur (52, 54), dans lequel le système de recueil-répartition supérieur est constitué de la ou des couches disposées entre la feuille de dessus et l'âme absorbante, dans lequel le système de recueil-répartition supérieur comprend au moins une couche lacée par filage et dans lequel ledit système de recueil-répartition supérieur est exempt de fibres de cellulose réticulée non liées ; et
- une feuille de fond imperméable aux liquides (25) ;

dans lequel les particules de polymère superabsorbant ont une mesure de perméabilité à l'urine (UPM) d'au moins $45,10^{-7}$ ($cm^3$.s)/g, telle que mesurée par le procédé UPM décrit ici.

2. Article absorbant selon la revendication 1, dans lequel les particules de polymère superabsorbant ont une UPM dans la plage de $55,10^{-7}$ à $90,10^{-7}$ ($cm^3$.s)/g.

3. Article absorbant selon la revendication 1 ou 2, dans lequel les particules de polymère superabsorbant ont une capacité efficace (EFFC) supérieure à 23 g/g, dans lequel la capacité efficace est mesurée comme décrit ici.

4. Article absorbant selon la revendication 3, dans lequel les particules de polymère superabsorbant ont une EFFC dans la plage allant de 23,5 g/g à 29 g/g.

5. Article absorbant selon l'une quelconque des revendications précédentes, dans lequel les particules de polymère superabsorbant ne sont pas mélangées avec des fibres de cellulose.

6. Article absorbant selon l'une quelconque des revendications précédentes, dans lequel le système de recueil-répartition supérieur comprend une première couche et une seconde couche, la première couche étant plus près de la feuille de dessus et la seconde couche étant plus près de l'âme absorbante, dans lequel la première couche est une couche de recueil non tissée (52) et la seconde couche est une couche de répartition non tissée (54).

7. Article absorbant selon la revendication 6, dans lequel la seconde couche (54) est un non-tissé lacé par filage.

**8.** Article absorbant selon l'une quelconque des revendications précédentes, comprenant en outre une couche d'acquisition et de répartition inférieure (56) entre la couche de particules de polymère superabsorbant et la feuille de fond, dans lequel la couche d'acquisition et de répartition inférieure comprend ou est constituée d'une couche de non-tissé, en particulier d'une couche de non-tissé cardée.

**9.** Article absorbant selon la revendication 8, dans lequel la couche d'acquisition et de répartition inférieure a une masse surfacique allant de 20 g/m$^2$ à 100 g/m$^2$, de préférence allant de 30 g/m$^2$ à 50 g/m$^2$.

**10.** Article absorbant selon l'une quelconque des revendications 8 à 9, dans lequel la couche d'acquisition et de répartition inférieure (56) comprend un revêtement tensioactif et/ou un additif de fusion hydrophile.

**11.** Article absorbant selon l'une quelconque des revendications 7 à 10, dans lequel la couche d'acquisition et de répartition inférieure (56) est disposée entre la couche d'enveloppe d'âme inférieure (46) et la feuille de fond (25), et dans lequel la couche d'acquisition et de répartition inférieure (56) et la couche d'enveloppe d'âme inférieure (46) sont l'une et l'autre hydrophiles, la couche d'acquisition et de répartition inférieure (56) étant moins hydrophile que la couche d'enveloppe d'âme inférieure (46).

**12.** Article absorbant selon l'une quelconque des revendications précédentes, dans lequel au moins certaines des particules de polymère superabsorbant sont immobilisées par un filet fibreux thermoplastique sur au moins l'une de la couche d'enveloppe d'âme supérieure (45) ou de la couche d'enveloppe d'âme inférieure (46).

**13.** Article absorbant selon l'une quelconque des revendications précédentes, dans lequel la couche de particules de polymère superabsorbant (60) comprend au moins un canal s'étendant longitudinalement (26) qui est exempt de particules de polymère superabsorbant.

**14.** Article absorbant selon l'une quelconque des revendications précédentes, dans lequel les particules de polymère superabsorbant sont obtenues par réticulation de surface d'un PSA précurseur, dans lequel le PSA précurseur est de préférence réticulé de façon interne.

Fig. 1

Fig. 2

Fig. 3A

Fig. 3B

Fig. 3C

Fig. 4

Fig. 5

Fig. 6

EP 4 489 711 B1

**Fig. 7**

Fig. 8

Fig. 9

**Fig. 10**

## REFERENCES CITED IN THE DESCRIPTION

**Patent documents cited in the description**

- US 20110125119 A1
- WO 9511654 A, Tanzer
- WO 2008155699 A1, Hundorf
- US 20080312622 A, Hundorf
- US 20210106471 A
- WO 2021118904 A, Grenier
- WO 2020205485 A, Peri
- US 3860003 A
- US 5221274 A
- US 5554145 A
- US 5569234 A
- US 5580411 A
- US 6004306 A
- US 5599335 A, Goldman
- EP 1447066 A, Busam
- WO 9511652 A, Tanzer
- US 20080312622 A1, Hundorf
- WO 2012052172 A, Van Malderen
- US 2006024433 A, Blessing
- US 20080312617 A
- US 20100051166 A1, Hundorf
- US 2008312622 A1, Hundorf
- WO 2012170778 A1
- WO 2012170781 A, Kreuzer
- WO 2012170798 A1, Jackels
- EP 2905000 A, Jackels
- EP 2905001 A, Armstrong-Ostle
- WO 2015041784 A1
- EP 2535027 A1
- US 4340706 A
- US 5849816 A
- US 20090192035 A
- US 20090258994 A
- US 20100068520 A
- WO 2006083584 A
- EP 530438 A
- EP 547847 A
- EP 559476 A
- EP 632068 A

- WO 9321237 A
- WO 03104299 A
- WO 03104300 A
- WO 03104301 A
- DE 10331450 A
- DE 10331456 A
- DE 10355401 A
- DE 19543368 A
- DE 19646484 A
- WO 9015830 A
- WO 0232962 A
- WO 2007047598 A
- WO 2007046052 A
- WO 2009155265 A
- WO 2009155264 A
- EP 3391961 A1, Kamphus, P&G
- US 5300565 A
- US 5180622 A, Berg
- US 5149334 A
- US 5102597 A, Roe
- US 5492962 A, Lahrman
- EP 3056521 B1, Kim
- EP 1512712 B1, Koji
- US 10414876 B2, Jang
- US 7429009 B2, Nagasawa
- EP 220224911 A, Higashimoto
- EP 2011803 B1, Handa
- EP 2944376 A1
- EP 3391963 A
- US 20210106471 A1, Yuan
- US 6146757 A
- US 20140143180 A1
- US 20070219521 A1, Hird
- US 20110139658 A1, Hird
- US 20110139657 A1, Hird
- US 20110152812 A1, Hird
- US 20110139662 A1, Hird
- US 20110139659 A1, Hird
- WO 2021188330 A

**Non-patent literature cited in the description**

- Contact Angle, Wettability and Adhesion. 1964
- Absorbency. Elsevier, 1982, 42-43

- Chemical Engineering. Pergamon Press, 1978, vol. II, 122-127